# EUROPEAN PATENT APPLICATION

(11) **EP 2 090 656 A1**
(43) Date of publication of application: **19.08.2009**
(21) Application number: 09075054.8
(22) Date of filing: 02.06.2003
(51) Int. Cl.: C12N 15/12, C12N 5/10

(54) **Mutant androgen receptor, cancer cells expressing the same, method of constructing the same and use thereof**

(30) Priority: 03.06.2002 JP 2002162206; 30.08.2002 JP 2002255612
(62) Divisional of application: 03730778.2
(71) Applicant: Takeda Pharmaceutical Company Limited, Osaka 541-0045 (JP)
(72) Inventor: Hara, Takahito, Osaka-shi Osaka 532-0013 (JP); Kusaka, Masami, Kobe-shi Hyogo 651-2102 (JP); Miyazaki, Junichi, Kobe-shi Hyogo 658-0073 (JP)
(74) Representative: Dey, Michael

(57) **Abstract**

The present invention provides a mutant androgen receptor (AR) useful for screening an agent for the prophylaxis/treatment of androgen-independent cancer and the like, DNA encoding the AR and the like. It also provides a method of producing a mutant AR expressing cancer cell line in vitro, a method of screening for an anti-antiandrogen drug using the mutant AR expressing cancer cell line obtained by this method, a method of classifying anti-androgen drugs, a cocktail therapy agent comprising a combination of anti-androgen drugs classified by this method and the like.

## Description

### Technical Field

The present invention relates to a mutant androgen receptor derived from human prostate cancer cells, a human prostate cancer cell line that expresses it, a method of producing them, a method of screening an antiandrogen drug that exhibits proliferation-suppressing action even on prostate cancer in the androgen-independent stage using them, and the like.

### Background Art

For advanced prostate cancer with metastases, it is standard practice worldwide to conduct androgen suppression endocrine therapy as the first choice. Also, in localized prostate cancer without metastases, it was demonstrated that prostate cancer progression is delayed by conducting an endocrine therapy with an antiandrogen drug after radical prostatectomy or radiotherapy; endocrine therapy is spreading for localized prostate cancer as well.

Androgens generically refer to steroid hormones possessing male sex hormone actions; testosterone, which is synthesized in the testis, dehydroepiandrosterone and androstenedione, which are synthesized in the adrenal cortex, and the like can be mentioned. Because about 95% of androgens are secreted from the testis, surgical (orchiectomy) or chemical (estrogen, LHRH agonist and the like) castration for blocking testis-derived testosterone is frequently used as androgen suppression endocrine therapy. However, because the adrenal-cortex-derived testosterone precursor is converted to highly active dihydrotestosterone (DHT) in prostate cells, it is sometimes not negligible as a proliferative stimulation to androgen-hypersensitive cancer cells, which are considered to exist at a given ratio in prostate cancer lesions, even if their abundance is apparently only several percent. For this reason, MAB (Maximal Androgen Blockade) therapy, in which an antiandrogen drug (particularly a non-steroidal drug) that antagonistically inhibits the binding of androgens to their receptors in the prostate is used in combination with castration surgery, is also conducted.

On the other hand, no effective prophylactic/therapeutic method for androgen-independent relapsed cancers, which are experienced sooner or later in almost all patients in the advanced stage undergoing endocrine therapy, has been developed. Although the mechanism of cancer relapse to endocrine therapy has not been elucidated, a plurality of factors, such as development/increase of cells that have acquired androgen hypersensitivity as a result of androgen receptor (AR) gene amplification or overexpression, and non-androgen substances (for example, glucocorticoids and antiandrogen drugs themselves) becoming acting as agonists due to AR mutations, are suggested.

For example, some clinical findings have been reported concerning the relationship between cancer relapse after antiandrogen drug administration and AR mutations. That is, AR mutations were observed in 5 of 17 patients who experienced relapsed prostate cancer after an endocrine therapy with a combination of flutamide and castration, all of which mutations were missense mutations of the 877th amino acid (corresponding to amino acid number 882 in the amino acid sequence of SEQ ID NO: 2) (Taplin et al., Cancer Res., 59: 2511-2515, 1999). On this type of mutant ARs, some antiandrogen drugs, including flutamide, conversely exhibited an action to stimulate cancer cell proliferation (Veldscholte et al., Biochem. Biophys. Res. Commun., 173: 534-540, 1990). Also, although missense mutations of AR were identified in 3 of 11 biopsy samples from patients who experienced relapsed prostate cancer after an endocrine therapy with a combination of bicalutamide and surgical castration, all mutation sites were other than the 877th amino acid, which is a prevalent mutation site in flutamide-resistant relapsed cancers (Haapala et al., Lab. Invest., 81: 1647-1651, 2001).

In developing a drug useful for the prophylaxis/treatment of androgen-independent cancers based on AR mutations, cancer cell lines that express such mutant ARs are very useful research tools. However, all mutant-AR-expressing cancer cells that have been reported to date were clinically obtained from relapsed cancers or pre-treatment prostate cancer; there is no case wherein a mutation was induced in AR-expressing cancer cells *in vitro* to establish a cancer cell line that expresses an AR having a mutation similar to those of clinically found mutant ARs derived from androgen-independent cancers.
Also, mutant ARs clinically found in androgen-independent cancers mostly have a single mutation at the amino acid level; reported cases of ARs having amino acid mutations at 2 sites or more are not unavailable but are very rare.

Accordingly, the object of the present invention is to provide a method of producing *in vitro* a cancer cell line that expresses an AR having a mutation similar to those of clinically found mutated ARs derived from androgen-independent cancers, and the mutant AR and the cancer cell line that expresses it which are obtained by said method, and to provide a method of screening a drug effective in the prophylaxis/treatment of androgen-independent relapsed cancers to endocrine therapy using them.

### Disclosure of the Invention

The present inventors conducted extensive investigations with the aim of accomplishing the above-described object, and found that when human prostate cells that are sensitive to a specified antiandrogen drug are cultured in the presence of said antiandrogen drug for a long period, a mutation to cause the antiandrogen drug to considerably lose potency or conversely exhibit agonist-like action is induced in an AR, and, as a result, the cells become an antiandrogen-drug-resistant, that is, androgen-independent, cancer and begin proliferation. Furthermore, the present inventors isolated the cDNA of the AR from cancer cells showing proliferation, analyzed the entire base sequence thereof, and found that the mutation site of the AR agrees well with that of a mutant AR clinically obtained from a relapsed cancer in a patient undergoing an endocrine therapy with an antiandrogen drug. The present inventors conducted further investigations based on these findings, which resulted in the completion of the present invention.

Accordingly, the present invention provides:
[1] a method of producing an antiandrogen-drug-resistant cancer cell line that expresses a mutant androgen receptor, which comprises culturing cancer cells sensitive to a specified antiandrogen drug in the presence of said antiandrogen drug, selecting a cancer cell line showing proliferation, analyzing the base sequence of the androgen receptor gene in said cancer cell line and selecting a line in which a mutation has occurred in said sequence;
[2] the method of the aforementioned [1], wherein the mutation site agrees with a clinical mutation site that appears due to administration of said antiandrogen drug;
[3] a method of producing an antiandrogen-drug-resistant cancer cell line that expresses a multiple-mutant androgen receptor, which comprises culturing cancer cells that express a mutant androgen receptor and are sensitive to a specified antiandrogen drug in the presence of said antiandrogen drug, selecting a cancer cell line showing proliferation, analyzing the base sequence of the mutant androgen receptor gene in said cancer cell line and selecting a line that has shown a different mutation in said sequence;
[4] the method of any one of the aforementioned [1] to [3], wherein the antiandrogen drug is flutamide or an analogue thereof or bicalutamide or an analogue thereof;
[5] a cancer cell line that expresses a mutant androgen receptor, which is obtained by the method of the aforementioned [1];
[6] a cancer cell line that expresses a multiple-mutant androgen receptor, which is obtained by the method of the aforementioned [3];
[7] the cancer cell line of the aforementioned [5] or [6], which further comprises a gene under the control of an androgen-responsive promoter that permits expression analysis;
[8] the cancer cell line of the aforementioned [7], wherein said gene is the prostate-specific antigen gene or a foreign reporter gene;
[9] a method of screening for an antiandrogen drug that exhibits antagonistic action on the mutant androgen receptor expressed in the cancer cell line of the aforementioned [5] or [6], which comprises culturing said cancer cell line in the presence of a test substance;
[10] a method of screening for an antiandrogen drug that exhibits antagonistic action on the mutant androgen receptor expressed in the cancer cell line of the aforementioned [7], which comprises culturing said cancer cell line in the presence of a test substance, and analyzing the expression of the gene under the control of the androgen-responsive promoter in said cancer cells;
[11] an antiandrogen drug that exhibits antagonistic action on a mutant androgen receptor, which is selected by the method of the aforementioned [9] or [10];
[12] a method of screening for an antiandrogen drug having no or little potential to induce resistant cancer, which comprises culturing cells of an androgen-sensitive cancer in the presence of a test substance and examining over time the appearance of a cancer cell line capable of proliferating under said conditions;
[13] an antiandrogen drug having no or little potential to induce resistant cancer, which is selected by the method of the aforementioned [12];
[14] the antiandrogen drug of the aforementioned [13], which has no or little potential to induce a mutation in an androgen receptor;
[15] an agent for the prophylaxis/treatment of hormone-sensitive cancers in the androgen-dependent stage or the androgen-independent stage, which comprises the antiandrogen drug of the aforementioned [11] or [13];
[16] a method for the prophylaxis/treatment of hormone-sensitive cancers in the androgen-dependent stage or the androgen-independent stage in a mammal, which comprises administering an effective amount of the antiandrogen drug of the aforementioned [11] or [13] to said mammal;
[17] use of the antiandrogen drug of the aforementioned [11] or [13], for the production of an agent for the prophylaxis/treatment of hormone-sensitive cancers in the androgen-dependent stage or the androgen-independent stage;
[18] the cancer cell line of the aforementioned [7] or [8], wherein the androgen-responsive promoter is a PSA promoter;
[19] the method of screening of the aforementioned [10], wherein the androgen-responsive promoter is a PSA promoter;
[20] a kit for evaluating antiandrogen drug responsiveness of transcription factor activity of an androgen receptor or for screening an androgen receptor modulator, which comprises as a component thereof mammalian cells containing a gene under the control of a PSA promoter that permits expression analysis;
[21] the kit of the aforementioned [20], wherein the mammalian cells further express a specified androgen receptor;
[22] the kit of the aforementioned [20], wherein a plurality of PSA promoters are tandemly ligated;
[23] the kit of the aforementioned [20], wherein the gene that permits expression analysis is a prostate-specific antigen gene or a foreign reporter gene;
[24] a method of evaluating antiandrogen drug responsiveness of transcription factor activity of an androgen receptor, which comprises bringing said androgen receptor and a specified antiandrogen drug into contact with mammalian cells containing a gene under the control of a PSA promoter that permits expression analysis, and analyzing the expression of said gene;
[25] a method of screening for a modulator of a specified androgen receptor, which comprises bringing said androgen receptor and a test substance into contact with mammalian cells comprising a gene under the control of a PSA promoter that permits expression analysis, and analyzing the expression of said gene;
[26] the method of the aforementioned [24] or [25], wherein the contact of the androgen receptor is accomplished by the expression of said receptor in the mammalian cells;
[27] the method of the aforementioned [24] or [25], wherein a plurality of PSA promoters is tandemly ligated;
[28] the method of the aforementioned [24] or [25], wherein the gene that permits expression analysis is the prostate-specific antigen gene or a foreign reporter gene;
[29] a protein comprising the same or substantially the same amino acid sequence as the following amino acid sequence (a) or (b):
   (a) an amino acid sequence in which tryptophan at amino acid number 746 is substituted by leucine in the amino acid sequence represented by SEQ ID NO: 2,
   (b) an amino acid sequence in which tryptophan at amino acid number is 746 substituted by leucine or cysteine and threonine at amino acid number 882 substituted by alanine in the amino acid sequence represented by SEQ ID NO: 2,
      or a salt thereof;
[30] a partial peptide of the protein of the aforementioned [29], which comprises at least a partial amino acid sequence corresponding to a region necessary for binding with an androgen in a normal androgen receptor, or an amide thereof or an ester thereof or a salt thereof;
[31] a polynucleotide comprising a polynucleotide that encodes the protein of aforementioned [29] or the partial peptide of the aforementioned [30];
[32] the polynucleotide of the aforementioned [31], which is a DNA;
[33] the polynucleotide of the aforementioned [31], which has the following base sequence (a) or (b):
   (a) a base sequence in which the base at base number 2237 is substituted by thymine in the base sequence represented by SEQ ID NO: 1,
   (b) a base sequence in which the base at base number 2237 or 2238 is substituted by thymine and the base at base number 2644 is substituted by guanine in the base sequence represented by SEQ ID NO: 1;
[34] a diagnostic agent comprising the polynucleotide of the aforementioned [31];
[35] the diagnostic agent of the aforementioned [34], which is for the diagnosis of hormone-independent cancers;
[36] a recombinant vector comprising the polynucleotide of the aforementioned [31];
[37] a transformant transformed with the recombinant vector of the aforementioned [36];
[38] an animal cell having an ability of to produce the protein of the aforementioned [29];
[39] the animal cell of the aforementioned [38], which is a cancer cell;
[40] the animal cell of the aforementioned [39], wherein the cancer cell is a cell derived from prostate cancer;
[41] a method of producing the protein or the salt thereof of the aforementioned [29], or the partial peptide or the amide thereof or the ester thereof or the salt thereof of the aforementioned [30], which comprises culturing the transformant of the aforementioned [37] or the animal cell of the aforementioned [38] to allow the same to produce the protein of the aforementioned [29] or the partial peptide of the aforementioned [30];
[42] a method of screening a compound that alters the bindability of an androgen and the protein or the salt thereof of the aforementioned [29] or the partial peptide or the amide thereof or the ester thereof or the salt thereof of the aforementioned [30], or a salt thereof, which comprises using the protein or the salt thereof of the aforementioned [29] or the partial peptide or the amide thereof or the ester thereof or the salt thereof of the aforementioned [30];
[43] a kit for screening a compound that alters the bindability of an androgen and the protein or the salt thereof of the aforementioned [29] or the partial peptide or the amide thereof or the ester thereof or the salt thereof of the aforementioned [30], or a salt thereof, which comprises the protein or the salt thereof of the aforementioned [29] or the partial peptide or the amide thereof or the ester thereof or the salt thereof of the aforementioned [30];
[44] an agent for the prophylaxis/treatment of hormone-sensitive cancers in the androgen-dependent stage and the androgen-independent stage, which comprises a combination of two or more kinds of antiandrogen drugs that exhibit anti-androgen action on different mutant androgen receptors;
[45] the agent of the aforementioned [44], wherein one of the two or more kinds of antiandrogen drugs is selected by the method of the aforementioned [9] or [10];
[46] a method for the prophylaxis/treatment of hormone-sensitive cancers in the androgen-dependent stage or the androgen-independent stage in a mammal, which comprises administering, to said mammal, an effective amount of each of two or more kinds of antiandrogen drugs that exhibit an antiandrogen action on different mutant androgen receptors;
[47] the method of the aforementioned [46], wherein one of the two or more kinds of antiandrogen drugs is selected by the method of the aforementioned [9] or [10];
[48] use of two kinds or more of antiandrogen drugs that exhibit an antiandrogen action on different mutant androgen receptors, for the production of an agent for the prophylaxis/treatment of hormone-sensitive cancers in the androgen-dependent stage or the androgen-independent stage;
[49] the use of the aforementioned [48], wherein one of the two or more kinds of antiandrogen drugs is selected by the method of the aforementioned [9] or [10];
[50] an antibody against the protein or the salt thereof of the aforementioned [29] or the partial peptide or the amide thereof or the ester thereof or the salt thereof of the aforementioned [30], which does not recognize a normal androgen receptor protein or a salt thereof;
[51] an antibody against a protein comprising the same or substantially the same amino acid sequence as an amino acid sequence in which tryptophan at amino acid number 746 is substituted by leucine or cysteine in the amino acid sequence represented by SEQ ID NO: 2, or a salt thereof, which does not recognize a protein comprising the same or substantially the same amino acid sequence as an amino acid sequence in which threonine at amino acid number 882 is substituted by alanine in the amino acid sequence represented by SEQ ID NO: 2, or a salt thereof;
[52] an antibody against a protein comprising the same or substantially the same amino acid sequence as an amino acid sequence in which threonine at amino acid number 882 is substituted by alanine in the amino acid sequence represented by SEQ ID NO: 2, or a salt thereof, which does not recognize a protein comprising the same or substantially the same amino acid sequence as an amino acid sequence in which tryptophan at amino acid number 746 is substituted by leucine or cysteine in the amino acid sequence represented by SEQ ID NO: 2, or a salt thereof;
[53] the antibody of the aforementioned [50], which is a neutralizing antibody that suppresses a transcription factor activity of the protein of the aforementioned [29];
[54] a diagnostic agent comprising the antibody of any one of the aforementioned [50] to [52];
[55] the diagnostic agent of the aforementioned [54], which is for the diagnosis of transition of hormone-sensitive cancers to the androgen-independent stage;
[56] a compound that alters the bindability of an androgen and the protein or the salt thereof of the aforementioned [29] or the partial peptide or the amide thereof or the ester thereof or the salt thereof of the aforementioned [30], or a salt thereof, which can be obtained using the method of screening of the aforementioned [42] or the kit for screening of the aforementioned [43];
[57] a pharmaceutical agent comprising the compound or the salt thereof of the aforementioned [56];
[58] the pharmaceutical agent of the aforementioned [57], which is an agent for the prophylaxis/treatment of hormone-sensitive cancers in the androgen-dependent stage and the androgen-independent stage;
[59] a method of quantitating the mRNA that encodes the protein of the aforementioned [29],which comprises using the polynucleotide of the aforementioned [31] or a portion thereof;
[60] a method of quantitating the protein or the salt thereof of the aforementioned [29] or the partial peptide or the amide thereof or the ester thereof or the salt thereof of the aforementioned [30], which comprises using the antibody of any one of the aforementioned [50] to [52];
[61] a diagnostic method for transition of hormone-sensitive cancers to the androgen-independent stage, which comprises using the quantitation method of the aforementioned [59] or [60];
[62] a method of classifying antiandrogen drugs, which comprises distinguishing antiandrogen drugs that permit generation of a resistant cancer cell line that expresses the same mutant androgen receptor by the method of the aforementioned [1], as one group, from a group consisting of antiandrogen drugs that permit generation of a resistant cancer cell line that expresses a different mutant androgen receptor;
[63] an agent for the prophylaxis/treatment of hormone-sensitive cancers in the androgen-dependent stage and the androgen-independent stage, which comprises a combination of two or more kinds of antiandrogen drugs classified under different groups by the method of the aforementioned [62];
[64] a method for the prophylaxis/treatment of hormone-sensitive cancers in the androgen-dependent stage or the androgen-independent stage in a mammal, which comprises administering, to said mammal, an effective amount of each of two or more kinds of antiandrogen drugs classified under different groups by the method of the aforementioned [62];
[65] use of two or more kinds of antiandrogen drugs classified into different groups by the method of the aforementioned [62], for the production of an agent for the prophylaxis/treatment of hormone-sensitive cancers in the androgen-dependent stage or the androgen-independent stage; and
[66] a method for the prophylaxis/treatment of hormone-sensitive cancers in the androgen-independent stage, which comprises administering, to a mammal in which a cancer resistant to an antiandrogen drug that falls under one of the groups classified by the method of the aforementioned [62] has developed due to long-term administration of an effective amount of said antiandrogen drug, an effective amount of an antiandrogen drug that is classified into a different group, and the like.

### Brief Description of the Drawings

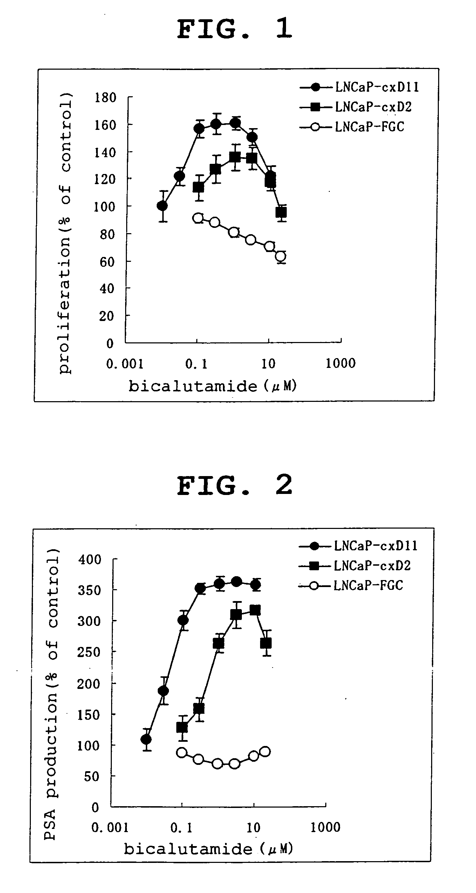

Figure 1 is a drawing showing the effects of bicalutamide on the proliferation of LNCaP-cxD11, LNCaP-cxD2 and LNCaP-FGC cells. LNCaP-cxD11, LNCaP-cxD2 and LNCaP-FGC cells suspended in a medium (RPMI1640 + 10% DCC-FBS) were sown to 24-well plates at 40000 cells/plate, and, on the following day, bicalutamide was added. Three days after bicalutamide addition, cells were counted. Mean ± standard error, n=3.
Figure 2 is a drawing showing the effects of bicalutamide on the PSA production by LNCaP-cxD11, LNCaP-cxD2 and LNCaP-FGC cells. LNCaP-cxD11, LNCaP-cxD2 and LNCaP-FGC cells suspended in a medium (RPMI1640 + 10% DCC-FBS) were sown to 24-well plates at 40000 cells/plate, and, on the following day, bicalutamide was added. Three days after bicalutamide addition, supernatant concentrations of PSA were measured. Mean ± standard error, n=3.

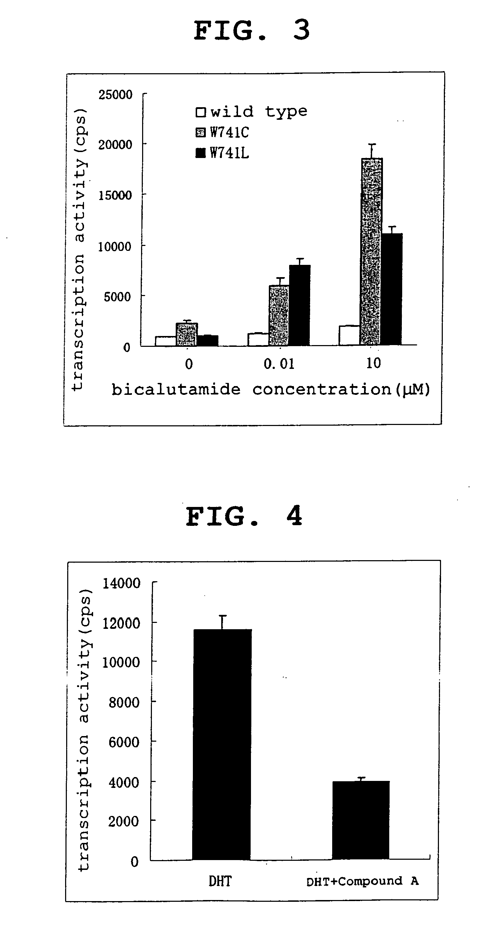

Figure 3 is a drawing showing the transcription-promoting activity of bicalutamide on wild type or mutant (W741C, W741L) ARs. A vector DNA incorporating each AR and a vector DNA having the luciferase gene linked downstream of an androgen-responsive promoter were co-transfected, and 0.01 or 1 µM bicalutamide was added, after which luciferase activity was measured. Mean ± standard error, n=4.
Figure 4 is a drawing showing the antagonistic action of Compound A on a W741C type mutant AR. A vector DNA incorporating the W741C type mutant AR and a vector DNA having the luciferase gene linked downstream of an androgen-responsive promoter were co-transfected, and 40nM DHT or both 40nM DHT and 10 µM Compound A were added, after which luciferase activity was measured. Mean ± standard error, n=16 for the only-DHT addition group and n=4 for the DHT+Compound A addition group.

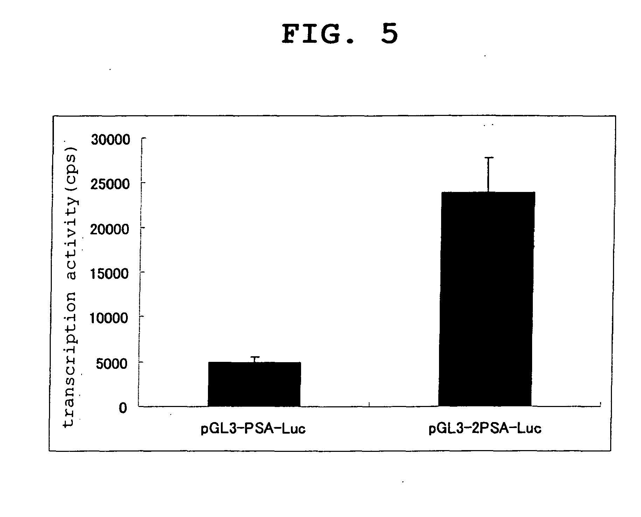

Figure 5 is a drawing showing the transcription activation of a gene under the control of the PSA promoter by a wild type AR activated by DHT. A vector DNA incorporating the wild type AR and a vector DNA having the luciferase gene linked downstream of one PSA promoter or two tandemly ligated PSA promoters were co-transfected to COS-7 cells, and 1 µM DHT was added, after which luciferase activity was measured. Mean ± standard error, n=6.

The mutant AR of the present invention is a protein containing the same or substantially the same amino acid sequence as an amino acid sequence in which tryptophan at amino acid number 746 is substituted by leucine in the amino acid sequence represented by SEQ ID NO: 2 (Proc. Natl. Acad. Sci. USA, 85: 7211-7215, 1988) (also abbreviated as "W746L"), or an amino acid sequence in which tryptophan at amino acid number 746 is substituted by leucine or cysteine and threonine at amino acid number 882 is substituted by alanine in the amino acid sequence represented by SEQ ID NO: 2 (also abbreviated as "W746L(C)+T882A").

The mutant AR of the present invention may be any protein derived from any cells (e.g., splenocytes, nerve cells, glial cells, beta cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, pituitary, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicle, testis, ovary, placenta, uterus, bone, joint, skeletal muscle, etc. (particularly prostate) from mammals (e.g., human, guinea pig, rats, mouse, rabbit, swine, sheep, bovine, monkey, etc.). The mutant AR may also be a synthetic protein.

The amino acid sequence, which is substantially the same amino acid sequence as W746L or W746L(C)+T882A includes an amino acid sequence having at least about 50% identity, preferably at least about 70% identity, more preferably at least about 80% identity, much more preferably at least about 90% identity and most preferably at least about 95% identity, to each pertinent amino acid sequence.

As examples of the protein containing substantially the same amino acid sequence as W746L or W746L(C)+T882A, a protein having substantially the same amino acid sequence as W746L or W746L(C)+T882A, and having the activity substantially equivalent to that of each of the pertinent amino acid sequences, and the like are preferred.

Examples of the substantially equivalent activity include a ligand (including, in addition to androgens, which are endogenous ligands, other steroid hormones, and agonists and antagonist such as antiandrogen drugs) binding activity, cell proliferation activity, and action to increase the expression of a gene under the control of an androgen-responsive promoter, such as PSA (prostate-specific antigen), and the like. "Substantially equivalent" indicates that the nature of the activities are the same. Therefore, although it is preferred that activities such as ligand binding activity, cell proliferation activity, action to increase the expression of a gene under the control of an androgen-responsive promoter, etc. be equivalent (e.g., about 0.01- to 100-fold, preferably about 0.5- to 20-fold, more preferably about 0.5- to 2-fold), quantitative factors such as the the degrees of these activities and the molecular weight of the protein may differ. These activities can be determined according to methods known per se; and also for example, according to the screening method described below.

More specifically, the activity substantially equivalent to W746L includes allowing bicalutamide or an analogue thereof to show an agonistic activity, that is, cell proliferation activity and PSA expression inducing activity in the presence of bicalutamide. Also, the activity substantially equivalent to W746L(C)+T882A includes allowing flutamide or an analogue thereof to show an agonistic activity, that is, cell proliferation activity and PSA expression inducing activity in the presence of flutamide, in addition to the same bicalutamide response as W746L.

Also, as the mutant AR of the present invention, a protein containing (1) an amino acid sequence in which 1 or more (preferably approximately 1 to 30, more preferably approximately 1 to 10, still more preferably several (1 to 5)) amino acids are deleted in W746L or W746L(C)+T882A, (2) an amino acid sequence in which 1 or more (preferably approximately 1 to 30, more preferably approximately 1 to 10, still more preferably several (1 to 5)) amino acids are added to W746L or W746L(C)+T882A, (3) an amino acid sequence in which 1 or more (preferably approximately 1 to 30, more preferably approximately 1 to 10, still more preferably several (1 to 5)) amino acids are substituted by other amino acids in W746L or W746L(C)+T882A, or (4) an amino acid sequence comprising a combination thereof (however, the 746th (and the 882nd) amino acid is conserved), and the like can also be used.

Throughout the present specification, proteins are represented in accordance with the conventional way of describing peptides, that is, the N-terminus (amino terminus) at the left hand side and the C-terminus (carboxyl terminus) at the right hand side. In the mutant AR of the present invention including W746L or W746L(C)+T882A, the C-terminus may be usually in the form of a carboxyl group (-COOH), a carboxylate (-COO⁻), an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, alpha -naphthyl, etc.; a C₄₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; and the like. In addition, pivaloyloxymethyl or the like, which is used widely as an ester for oral administration, may also be used.

When the mutant AR of the present invention has a carboxyl group (or a carboxylate) at a position other than the C-terminus, it may be amidated or esterified and such an amide or ester is also included within the mutant AR of the present invention. In this case, as examples of the ester, the above-described ester at the C-terminus and the like can be used.

Furthermore, examples of the mutant AR of the present invention include, referring to the above-described protein, those wherein the amino group of the methionine residue at the N-terminus is protected by a protecting group (for example, a C₁₋₆ acyl group such as a C₂₋₆ alkanoyl group such as a formyl group or acetyl, and the like), those wherein the glutamyl group resulting from cleavage on the N-terminus side *in vivo* is pyroglutamated, those wherein a substituent on the side chain of an amino acid in the molecule (for example, -OH, -SH, amino group, imidazole group, indole group, guanidino group and the like) is protected by an appropriate protecting group (for example, a C₁₋₆ acyl group such as a C₂₋₆ alkanoyl group such as a formyl group or acetyl, and the like); or complex proteins such as what is called a glycoprotein, wherein a sugar chain is bound, and the like.

Specific examples of the mutant AR of the present invention include a human-derived (more preferably human prostate cancer-derived) AR containing W746L or W746L(C)+T882A and the like.

As partial peptides of the mutant AR of the present invention (hereinafter sometimes referred to as the partial peptides of the present invention), those having a partial amino acid sequence, in the molecule of the mutant AR of the present invention, which corresponds to a region necessary for the binding of normal AR (the amino acid sequence represented by SEQ ID NO: 2 and polymorphism thereof) to androgen can be used. AR, like other nuclear receptors, has a transcription activation domain, a DNA binding domain, a hinge region and a ligand binding domain arranged in that order from the N-terminus side, the ligand binding domain being an amino acid sequence shown by amino acid numbers 672 to 924 in the amino acid sequence represented by SEQ ID NO: 2.

Although the number of amino acids in the partial peptide of the present invention is not subject to limitation, as long as it has a partial amino acid sequence necessary for the binding of a normal AR to androgen, a peptide having a sequence consisting of at least 20, preferably 50 or more, still more preferably 100 or more amino acids, and the like are preferred.

Substantially the same amino acid sequence includes an amino acid sequence having at least about 50% homology, preferably at least about 70% homology, more preferably at least about 80% homology, much more preferably at least about 90% homology and most preferably at least about 95% homology, to these amino acid sequences.

Herein, "activity substantially equivalent" refers to the same significance as defined above. The "activity substantially equivalent" can be assayed in the same manner as given above.

Also, the partial peptide of the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; or, in which at least 1 or 2 amino acids (preferably approximately 1 to 10 amino acids, more preferably several and most preferably approximately 1 to 5 amino acids) are substituted by other amino acids (provided that the 746th (and 882nd) amino acids are conserved).

In the partial peptide of the present invention, the C-terminus may be normally a carboxyl group (-COOH), carboxylate (-COO⁻), amide (-CONH₂) or an ester (-COOR). Here, R in the ester has the same definition as that given above.

When the partial peptide of the present invention has a carboxyl group (or a carboxylate) at a position other than the C-terminus, it may be amidated or esterified and such an amide or ester is also included within the partial peptide of the present invention. In this case, as examples of the ester, the above-described ester at the C-terminus and the like can be used.

As in the mutant AR of the present invention described above, the partial peptide of the present invention further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycopeptides, to which sugar chains are bound, and the like.

For salts of the mutant AR or the partial peptide of the present invention, preferred are salts with physiologically acceptable acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The mutant AR of the present invention or a salt thereof may be produced by isolating and purifying from animal cells having an ability of producing said mutant AR using a publicly known method for purifying a receptor protein. For example, it can be purified and isolated by homogenizing human or mammalian tissues followed by extraction with an acid or the like, and subjecting the extract to a combination of chromatography techniques such as reverse phase chromatography, ion exchange chromatography and the like.

Examples of animal cells having an ability of producing the mutant AR of the present invention include the aforementioned human or mammalian cells or tissues (particularly cells derived from a relapsed cancer of a prostate cancer patient receiving an antiandrogen drug (e.g., bicalutamide, or bicalutamide and flutamide)), an antiandrogen drug-resistant cancer cell line obtained by culturing a cancer cell line that expresses a normal AR or an AR having the T882A mutation (e.g., LNCaP cell line) in the presence of an antiandrogen drug (e.g., bicalutamide or an analogue thereof) followed by selection using cell proliferation as an index, and the like.

The mutant AR of the present invention or a salt thereof may also be produced by culturing a transformant transformed with a recombinant vector that contains a DNA encoding the mutant AR of the present invention described below. Alternatively, it may also be produced by the protein synthesis methods to be hereinafter described or modifications thereof, based on the amino acid sequence of W746L or W746L(C)+T882A.

To synthesize the mutant AR of the present invention, its partial peptide, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethozyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, tertiary butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. Both proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The mutant AR of the present invention or the partial peptide of the present invention (hereinafter "the partial peptide of the present invention or an amide thereof or an ester thereof or a salt thereof" is also referred to as "the partial peptide of the present invention", and "the mutant AR of the present invention or a salt thereof" is also referred to as "the mutant AR of the present invention") can be produced by publicly known methods for peptide synthesis, or by cleaving the mutant AR of the present invention or a protein containing the mutant AR of the present invention with an appropriate peptidase. For the methods for peptide synthesis, for example, either solid phase synthesis or liquid phase synthesis may be used. That is, the partial peptide or amino acids that can construct the mutant AR of the present invention or the partial peptide of the present invention are condensed with the remaining part. Where the product contains protecting groups, these protecting groups are removed to give the desired peptide. Publicly known methods for condensation and elimination of the protecting groups are described in (1)-(5) below:
(1) M. Bodanszky & M.A. Ondetti: Peptide Synthesis, Interscience Publishers, New York (1966);
(2) Schroeder & Luebke: The Peptide, Academic Press, New York (1965);
(3) Nobuo Izumiya, et al.: Peptide Gosei-no-Kiso to Jikken (Basics and experiments of peptide synthesis), published by Maruzen Co. (1975);
(4) Haruaki Yajima & Shunpei Sakakibara: Seikagaku Jikken Koza (Biochemical Experiment) 1, Tanpakushitsu no Kagaku (Chemistry of Proteins) IV, 205 (1977);
(5) Haruaki Yajima, ed.: Zoku Iyakuhin no Kaihatsu (A sequel to Development of Pharmaceuticals), Vol. 14, Peptide Synthesis, published by Hirokawa Shoten.

Also, after completion of the reaction, the mutant AR of the present invention or the partial peptide of the present invention may be purified and isolated using a combination of conventional purification methods such as solvent extraction, distillation, column chromatography, liquid chromatography, recrystallization and the like. When the mutant AR of the present invention or the partial peptide of the present invention obtained by the above methods is in a free form, it can be converted into an appropriate salt by a publicly known method; when it is obtained in a salt form, it can be converted into a free form by a publicly known method.

The polynucleotide encoding the mutant AR of the present invention may be any polynucleotide so long as it contains the base sequence (DNA or RNA, preferably DNA) encoding the mutant AR of the present invention described above. Such a polynucleotide may also be any one of DNA encoding the mutant AR of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the mutant AR of the present invention, mRNA of the mutant AR of the present invention can be quantified by, for example, the publicly known method published in separate volume of Jikken Igaku 15 (7) "New PCR and its application" (1997) and the like, or by its modifications.

The DNA encoding the mutant AR of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the mutant AR of the present invention may be any DNA, as long as it is a DNA having a base sequence in which the base at base number 2237 is substituted by thymine in the base sequence represented by SEQ ID NO: 1 (G2237T) or a base sequence in which the base at base number 2237 or 2238 is substituted by thymine and the base at base number 2644 is substituted by guanine in the base sequence represented by SEQ ID NO: 1 (G2237 (2238) T+A2644G), or a DNA having a base sequence that hybridizes to said base sequence under highly stringent conditions and encoding a mutant AR having the activities substantially equivalent to those of the mutant AR of the present invention (e.g., ligand binding activity, cell proliferation activity, PSA (prostate-specific antigen) increasing action and the like).

Examples of the DNA hybridizable to G2237T or G2237 (2238) T+A2644G include a DNA containing a base sequence having at least about 70% identity, preferably at least about 80% identity, more preferably at least about 90% identity and most preferably at least about 95% identity, to each of the base sequences.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

The polynucleotide comprising a part of the base sequence of the DNA encoding the mutant AR of the present invention or a part of the base sequence complementary to the DNA is used to mean to embrace not only the DNA encoding the partial peptide of the present invention described below but also RNA.

According to the present invention, antisense nucleotides (nucleic acids) that can inhibit the replication or expression of the mutant AR gene of the present invention can be designed and synthesized based on the base sequence information of the cloned or determined DNA encoding the mutant AR of the present invention. Such a nucleotide (nucleic acid) is specifically hybridizable to RNA of the mutant AR gene of the present invention by precisely controlling temperature conditions and the like to inhibit the synthesis or function of said RNA or capable of modulating or controlling the expression of the mutant AR gene of the present invention via interaction with an RNA associated with the mutant AR of the present invention. Polynucleotides complementary to the selected sequences of RNA associated with the AR of the present invention and nucleotides specifically hybridizable to the RNA associated with the mutant AR of the present invention are useful in modulating or controlling the expression of the mutant AR gene of the present invention *in vivo* and *in vitro,* and useful in treating diseases, particularly hormone-sensitive cancers (e.g., prostate cancer) in the androgen-dependent stage and the androgen-independent stage or in diagnosing their transition (relapse) to the androgen-independent stage.

The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the mutant AR gene of the present invention, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the mutant AR gene.

The relationship between the targeted nucleic acids and the nucleotides complementary to at least a part of the target, that is, the relationship between the target and the nucleotides hybridizable to the target, can be denoted to be "antisense". Examples of the antisense nucleotides include deoxynucleotides containing 2-deoxy-D-ribose, nucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense nucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory activity of the antisense nucleic acid can be examined using the transformant of the present invention, the gene expression system of the present invention *in vivo* and *in vitro,* or the translation system of the mutant AR of the present invention *in vivo* and *in vitro.*

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) DNA having a partial base sequence of the DNA having G2237T or G2237 (2238) T+A2644G, or (2) any DNA containing a partial base sequence of the DNA having a base sequence hybridizable to each of the base sequences under highly stringent conditions and encoding a mutant AR which has the activities (e.g., a ligand-biding activity, cell proliferation activity, PSA (prostate cancer specific antigen) increasing action) substantially equivalent to those of the partial peptide of the present invention.

Examples of the DNA that is hybridizable to G2237T or G2237 (2238) T+A2644G include DNA containing a base sequence having at least about 70% homology, preferably at least about 80% homology, more preferably at least about 90% homology and most preferably at least about 95% homology, to each of the base sequences.

For cloning of the DNA that completely encodes the mutant AR of the present invention or its partial peptide (hereinafter these are sometimes simply referred to as the mutant AR of the present invention), the DNA may be either amplified by PCR method using synthetic DNA primers containing a part of the base sequence encoding the mutant AR of the present invention, or selected by hybridizing the DNA inserted into an appropriate vector with a labeled DNA fragment or synthetic DNA that encodes a part or entire region of the mutant AR of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the Gapped duplex method or the Kunkel method or its modification by using a publicly known kit available as Mutan^{™}-G or Mutan^{™}-K (both manufactured by Takara Shuzo Co., Ltd.).

The cloned DNA encoding the mutant AR can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the mutant AR of the present invention can be manufactured, for example, by (a) excising the desired DNA fragment from the DNA encoding the mutant AR of the present invention, and then (b) ligating the DNA fragment with an appropriate expression vector downstream a promoter in the vector.

Examples of the vector include plasmids derived form E. coli (e.g., pBR322, pBR325, pUCl2, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SR α promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λP_{L} promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary, a signal sequence that matches with a host is added to the N-terminus of the AR of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the mutant AR of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DHl (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five^{™} cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of silkworm can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the AR can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogen phosphate, magnesium chloride, etc. In addition, yeast extract, vitamins, growth promoting factors etc. may also be added to the medium. Preferably, pH of the medium is adjusted to about 5 to about 8.

A preferred example of the medium for cultivation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultivated generally at about 30°C to about 40°C for about 6 hours to about 24 hours. If necessary, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the culture is performed at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium *(*Proceeding of the Society for the Biological Medicine, 73, 1 (1950)), etc. Preferably, pH of the medium is adjusted to about 6 to about 8. The culture is usually done at about 30°C to about 40°C for about 15 hours to about 60 hours and, if necessary, the culture can be aerated or agitated.

As described above, the mutant AR of the present invention can be produced within the cell or out of the cell of the transformant.

The mutant AR of the present invention can be separated and purified from the culture described above by the following procedures.

When the mutant AR of the present invention is extracted from the cultured bacteria or cells, after cultivation these cells are collected by a publicly known method and suspended in a appropriate buffer. The bacteria or cells are then disrupted by publicly known methods such as ultrasonication, a treatment with lysozyme and/or freeze-thaw cycling, followed by centrifugation, filtration, etc. Thus, the crude extract of the mutant AR of the present invention can be obtained. The buffer used for the procedures may contain a protein modifier such as urea or guanidine hydrochloride, or a surfactant such as Triton X-100^{™}, etc. When the mutant AR of the present invention is secreted in the culture, after completion of the cultivation the supernatant can be separated from the bacteria or cells to collect the supernatant by a publicly known method.

The AR of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the mutant AR of the present invention thus obtained is in a free form, it can be converted into the salt form by publicly known methods or modifications thereof. On the other hand, when the mutant AR is obtained in the salt form, it can be converted into the free form or in the form of a different salt by publicly known methods or modifications thereof.

The mutant AR of the present invention produced by the recombinant can be treated, prior to or after the purification, with an appropriate protein modifying enzyme so that the mutant AR can be appropriately modified and partially removed a polypeptide. Examples of the protein-modifying enzyme include trypsin, chymotrypsin, arginyl endopeptidase, protein kinase, glycosidase or the like.

The activity of the thus produced mutant AR of the present invention or salts thereof can be determined by a binding experiment to a labeled ligand, by an enzyme immunoassay using the antibody of the present invention (described in detail below) and the like.

Antibodies against the mutant AR of the present invention may be any of polyclonal antibodies and monoclonal antibodies, as long as they are capable of recognizing the AR of the present invention.

The antibodies against the mutant AR of the present invention may be manufactured by publicly known methods for manufacturing antibodies or antisera, using the mutant AR of the present invention as an antigen.

The antibody of the present invention is characterized in that it specifically recognizes the mutant AR of the present invention but does not recognize a normal AR. Accordingly, as the antigen used, a peptide containing a partial amino acid sequence of SEQ ID NO: 2 that is located at the amino acid number 746 and the vicinity thereof, and/or a partial amino acid sequence of SEQ ID NO: 2 that is located at the amino acid number 882 and the vicinity thereof, is preferred.

Another antibody of the present invention is an antibody that specifically recognizes a protein containing the same or substantially the same amino acid sequence as an amino acid sequence in which tryptophan at amino acid number 746 is substituted by leucine or cysteine in the amino acid sequence represented by SEQ ID NO: 2 (W746L(C)), or a salt thereof, but that does not recognize a protein containing the same or substantially the same amino acid sequence as an amino acid sequence in which threonine at amino acid number 882 is substituted by alanine in the amino acid sequence represented by SEQ ID NO: 2 (T882A), or a salt thereof. Still another antibody of the present invention is an antibody that specifically recognizes a protein containing the same or substantially the same amino acid sequence as T882A or a salt thereof, but that does not recognize a protein containing the same or substantially the same amino acid sequence as W746L(C) or a salt thereof. These antibodies can be prepared using, as an antigen, a partial amino acid sequence of the mutant AR recognized by them that is located at the mutation site and the vicinity thereof.

### [Preparation of monoclonal antibody]

### (a) Preparation of monoclonal antibody-producing cells

The mutant AR of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the mutant AR, which will be described later, with the antiserum followed by assaying the binding activity of the labeling agent bound to the antibody. The fusion may be operated, for example, by the known Koehler and Milstein method (*Nature, 256*, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the mutant AR of the present invention as an antigen directly or together with a carrier, adding an anti-immunoglobulin antibody (when mouse cells are used for the cell fusion, antimouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the mutant AR labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, a complex of immunogen (the mutant AR of the present invention as an antigen) and a carrier protein is prepared, and a mammal is immunized with the complex in a manner similar to the method described above for the manufacture of monoclonal antibodies. The product containing the antibody to the mutant AR of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

A DNA that encodes the mutant AR of the present invention or a salt thereof, a partial peptide thereof or an amide thereof or an ester thereof or a salt thereof can be used for (1) an agent for the prophylaxis and/or treatment of diseases that can be ameliorated by overexpression of the mutant AR of the present invention, (2) a genetic diagnostic agent, (3) a method of screening for a compound that alters the expression level of the mutant AR of the present invention or a salt thereof, (4) an agent for the prophylaxis and/or treatment of various diseases, which comprises a compound that alters the expression level of the mutant AR of the present invention, (5) a method of screening for a compound that alters the bindability of the mutant AR of the present invention and ligand (agonist, antagonist and the like), (6) an agent for the prophylaxis and/or treatment of various diseases, which comprises a compound that alters the bindability of the mutant AR of the present invention and ligand (agonist, antagonist), (7) quantitation of the mutant AR of the present invention, (8) a pharmaceutical agent comprising the antibody of the present invention, (9) a pharmaceutical agent comprising an antisense DNA, (10) production of a DNA-introduced animal, and the like.

Particularly, by a screening with a receptor binding assay system using an animal cell capable of expressing the mutant AR of the present invention or an expression system of a recombinant mutant AR, a compound that alters the bindability of a ligand for a human- or mammal-specific receptor (e.g., agonist, antagonist and the like) can be selected, and said agonist or antagonist can be used as an agent for the prophylaxis/treatment of various diseases, and the like.

The intended uses of the mutant AR of the present invention, the DNA that encodes the mutant AR of the present invention (hereinafter also abbreviated as the DNA of the present invention), and the antibody against the mutant AR of the present invention (hereinafter also abbreviated as the antibody of the present invention), are specifically described below.

### (1) Prophylactic and/or therapeutic agent for diseases that can be ameliorated by overexpression of the mutant AR of the present invention

The mutant AR of the present invention is characterized in that bicalutamide or an analogue thereof, or further flutamide or an analogue thereof, acts thereon as an agonist. Because it is known that in prostate cancer, a stimulation of AR in excess conversely suppresses proliferation, by locally delivering (1) the mutant AR of the present invention or (2) the DNA of the present invention to cancer cells and allowing it in excess therein, it is possible to suppress the proliferation of antiandrogen drug-resistant cancer cells.

When the mutant AR of the present invention is used as the above-described prophylactic/therapeutic agent, it can be made into a preparation in a conventional manner.

On the other hand, where the DNA of the present invention is used as the above-described prophylactic/therapeutic agent, the DNA of the present invention itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, (1) the mutant AR of the present invention or b) the DNA of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing (1) the mutant AR of the present invention or (2) the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80^{™} and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the mutant AR of the present invention varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (2) Genetic diagnostic reagent

By using the DNA of the present invention as a probe, the DNA or mRNA encoding the mutant AR of the present invention (i.e., an abnormality in a normal AR gene or mRNA) in mammals (e.g., human, rat, rabbit, sheep, swine, bovine, cat, dog, monkey and the like) can be detected. Therefore, it is useful as a genetic diagnostic agent for a cancer, particularly as a genetic diagnostic agent for the development, increase and the like of a cancer cell that has acquired resistance to an antiandrogen drug (e.g., bicalutamide, flutamide and the like).

The genetic diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States ofAmerica, 86, 2766-2770 (1989)).

### (3) Method of screening for a compound that alters the expression level of the mutant AR of the present invention

By using as a probe, the DNA of the present invention can be used for screening for a compound that alters the expression level of the mutant AR of the present invention.

That is, the present invention provides a method of screening for a compound that alters the expression level of the mutant AR of the present invention, which comprises measuring the amount of mRNA encoding the mutant AR of the present invention contained in, for example, (i) (1) blood, (2) specific organs, (3) tissues or cells isolated from the organs of non-human mammals (e.g., rat, rabbit, sheep, swine, bovine, cat, dog, monkey and the like), or in (ii) transformants, etc.

The amount of mRNA encoding mutant AR of the present invention can be specifically measured as follows.
(i) A non-human mammal (e.g., mouse, rat, rabbit, sheep, swine, bovine, cat, dog, monkey and the like) having cells that express a normal AR or the mutant AR of the present invention receive administration of a drug or physical stress, and blood, specific organs, or tissues or cells isolated from the organs are obtained after a specified period of time. The mRNA encoding the mutant AR of the present invention contained in the thus obtained cells is extracted from the cells, for example, in a conventional manner and quantified using, e.g., TaqManPCR, or may also be analyzed by Northern blot technique by publicly known methods.
(ii) A Transformant that expresses the mutant AR of the present invention or an animal cell capable of expressing the mutant AR of the present invention is prepared according to the methods described above, and the mRNA encoding the AR of the present invention can be quantified and analyzed in the same manner.

Screening for a compound that alters the expression level of the mutant AR of the present invention can be coducted by the following procedures:
(i) To a non-human mammal having cells that express a normal AR or the mutant AR of the present invention, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA encoding the mutant AR of the present invention contained in the cells is quantified and analyzed; or
(ii) A test compound is mixed in a culture medium and a transformant or an animal cell capable of expressing the mutant AR of the present invention is cultured therein in a conventional manner. After a specified time (after 1 day to 7 days, preferably after 1 day to 3 days, more preferably after 2 to 3 days), the amount of mRNA encoding the mutant AR of the present invention contained in the transformant or animal cell capable of expressing the mutant AR of the present invention can be quantified and analyzed.

The compound or its salt, which is obtainable by the screening method of the present invention, is a compound that alters the expression level of the mutant AR of the present invention, specifically, (a) a compound that excessively enhances a cell-stimulating activity (e.g., cell proliferation stimulation, PSA expression induction activity and the like) mediated by the mutant AR of the present invention by increasing the expression level of the mutant AR of the present invention, or (b) a compound that attenuates said cell-stimulating activity by decreasing the expression level of the mutant AR of the present invention.

The compound includes a peptide, protein, non-peptide compound, synthetic compound, and fermentation product. It may be a novel or known compound.

The compound that excessively enhances the cell-stimulating activity is capable of suppressing the proliferation of antiandrogen drug-resistant cancers by, for example, using it in combination with an antiandrogen drug, and is useful as a prophylactic/therapeutic agent for hormone-sensitive cancers (e.g., prostate cancer and the like) in the androgen-independent stage.

Since the compound that attenuates the cell-stimulating activity decreases the physiological activity of the mutant AR of the present invention, it is useful as a prophylactic/therapeutic agent for hormone-sensitive cancers (e.g., prostate cancer and the like) in the androgen-independent stage.

When the compound or its salt obtained by the screening method of the present invention is used as a pharmaceutical composition, the compound can be prepared into a pharmaceutical composition in a conventional manner. For example, in the same manner as the above-described pharmaceutical agent containing the mutant AR of the present invention, the compound can be made into tablets, capsules, elixirs, microcapsules, sterile solutions, suspensions and the like.

Since the thus obtained preparation is safe and low toxic, it can be administered to mammals (e.g., human, rat, rabbit, sheep, swine, bovine, cat, dog, monkey, etc.).

The dose of the compound or its salt varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (4) Prophylactic and/or therapeutic agent for various diseases, which comprises a compound that alters the expression level of the mutant AR of the present invention

A compound that alters the expression level of the mutant AR of the present invention can be used as a prophylactic/therapeutic agent for hormone-dependent and hormone-independent cancer (e.g., prostate cancer and the like). Said compound can be made into a preparation in a conventional manner.

For example, said compound can be used orally in the form of tablets which may be sugar coated if necessary, capsules, elixirs, microcapsules etc., or parenterally in the form of injectable preparations such as a sterile solution and a suspension in water or with other pharmaceutically acceptable liquid. For example, these preparations can be manufactured by mixing said compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80^{™} and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (5) Method of screening for a compound (agonist, antagonist and the like) that alters the bindability of the mutant AR of the present invention and a ligand

By using the mutant AR of the present invention, or by constructing an expression system for the recombinant mutant AR and using a receptor binding assay system with the above expression system, a compound that alters the bindability of a ligand and the mutant AR of the present invention (e.g., peptide, protein, non-peptide compound, synthetic compound, fermentation product, etc.), or a salt thereof, can be screened efficiently.

Such compounds include (a) a compound that has an activity to promote a cell-stimulating activity (e.g., cell proliferation activity, PSA expression induction activity and the like) via the mutant AR of the present invention (so-called agonist for the mutant AR of the present invention), (b) a compound that has no cell-stimulating activity (so-called antagonist for the mutant AR of the present invention), (c) a compound that enhances the binding force of a ligand and the mutant AR of the present invention, or (d) a compound that decreases the binding force of a ligand and the mutant AR of the present invention and the like.

That is, the present invention provides a method of screening for a compound that alters the bindability of a ligand and the mutant AR of the present invention or a salt thereof, which comprises comparing (i) the case wherein the mutant AR of the present invention is brought into contact with a ligand, with (ii) the case wherein the mutant AR of the present invention is brought into contact with a ligand and a test compound.

The screening method of the present invention is characterized by comprising determining, for example, the amount bound of ligand (androgen) for the AR of the present invention, a cell-stimulating activity (e.g., cell proliferation activity, PSA expression induction activity) and the like, in cases (i) and (ii), and comparing both cases.

More specifically, the present invention provides:
(1) a method of screening for a compound that alters the bindability of a ligand and the mutant AR of the present invention, or a salt thereof, which comprises measuring the amounts of a labeled ligand bound to the mutant AR of the present invention, when the labeled ligand is brought into contact with said mutant AR and when the labeled ligand and a test compound are brought into contact with said mutant AR, and comparing the both amounts,
(2) a method of screening for a compound that alters the bindability of a ligand and the mutant AR of the present invention, or a salt thereof, which comprises measuring the amounts of labeled ligand bound to cells containing the mutant AR of the present invention (animal cells capable of expressing the mutant AR, and the like), when the labeled ligand is brought into contact with said cells and when the labeled ligand and a test compound are brought into contact with said cells, and comparing the both amounts,
(3) a method of screening for a compound that alters the bindability of a ligand and the mutant AR of the present invention, or a salt thereof, which comprises measuring the amounts of labeled ligand bound to the mutant AR, when the labeled ligand is brought into contact with the mutant AR expressed by culturing a transformant containing the DNA of the present invention and when the labeled ligand and a test compound are brought into contact with the mutant AR of the present invention expressed by culturing a transformant containing the DNA of the present invention, and comparing the both amounts, and
(4) a method of screening for a compound that alters the bindability of a ligand and the mutant AR of the present invention, or a salt thereof, which comprises measuring receptor-mediated cell-stimulating activities (e.g., cell proliferation activity, PSA expression induction activity and the like), when a compound that activates the mutant AR of the present invention (e.g., an androgen or an antiandrogen drug such as bicalutamide or flutamide, and the like) is brought into contact with cells containing the mutant AR of the present invention (animal cells capable of expressing the mutant AR) and when the compound that activates the mutant AR of the present invention and a test compound are brought into contact with said cells, and comparing the both activities.

Hereinafter, the screening method of the present invention is described specifically.

First, for the mutant AR of the present invention used for the screening method of the present invention, any one may be used so long as it contains the mutant AR of the present invention described above. The aforementioned animal cells capable of expressing the mutant AR of the present invention, and the like are preferred.

To manufacture the mutant AR of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the mutant AR of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRa promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening method of the present invention, as a material that contains the mutant AR of the present invention, the mutant AR purified by publicly known methods or cells containing the mutant AR may be used.

In the screening method of the present invention, when cells containing the mutant AR of the present invention are used, the cells may be fixed with glutaraldehyde, formalin, etc. The cells can be fixed by publicly known methods.

The cells containing the mutant AR of the present invention are host cells that express said mutant AR. For the host cells, *Escherichia coli, Bacillus subtilis*, yeast, insect cells, animal cells and the like are preferred.

The amount of the mutant AR in the cells containing the mutant AR is preferably 10³ to 10⁸ molecules per cell, more preferably 10⁵ to 10⁷ molecules per cell. As the amount of expression increases, the ligand binding activity (specific activity) increases so that not only the highly sensitive screening system can be constructed but also large quantities of samples can be assayed with the same lot.

To perform the above-mentioned (1) to (3) to screen for a compound that alters the bindability of a ligand and the mutant AR of the present invention, for example, an appropriate mutant AR fraction and a labeled ligand are necessary.

The mutant AR fraction is preferably an AR fraction derived from cells expressing the mutant AR, which is obtained by a spontaneous or artificial mutation, or a recombinant mutant AR fraction having an activity equivalent thereto. Herein, equivalent activity is intended to mean an equivalent ligand binding activity, cell-stimulating activity or the like.

For the labeled ligand, a labeled ligand (androgen) and a labeled ligand analogue are used. For example, testosterone, DHT and the like labeled with [³H], [¹²⁵I], [¹⁴C], [³⁵S], etc. are used.

Specifically, to screen a compound that alters the bindabiliy of a ligand and the mutant AR of the present invention, first, the mutant AR standard is prepared by suspending cells containing the mutant AR of the present invention in a buffer appropriate for the screening. For the buffer, any buffer that does not interfere with the binding of the ligand to the mutant AR is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80^{™} (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and 10⁻⁴ M - 10⁻¹⁰ M of a test compound is simultaneously added to be co-present. To examine non-specific binding (NSB), a reaction tube containing an unlabeled test compound in large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance (B₀) as 100%, when the amount of specific binding (B-NSB) is, for example, 50% or less, the test compound can be selected as a candidate substance having a potential of competitive inhibition.

Of the method of (4) above to screen for a compound that alters the bindability of a ligand and the mutant AR of the present invention, the most preferable mode is described in detail below.

Generally, the cells containing the mutant AR of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the extent of proliferation of the cells is evaluated, or the cells are extracted or the supernatant is recovered and the resulting product is quantified by appropriate procedures. Where it is difficult to detect the production of the index substance (e.g., PSA and the like) for the cell-stimulating activity due to a degrading enzyme contained in the cells, an inhibitor against such a degrading enzyme may be added prior to the assay.

Screening by assaying the cell-stimulating activity requires cells that have expressed an appropriate mutant AR. For the cells that have expressed the mutant AR of the present invention, cells expressing the mutant AR, which is obtained by a spontaneous or artificial mutation, the cell line expressing the recombinant mutant AR described above and the like are desirable.

The test compound includes peptide, protein, non-peptide compound, synthetic compound, fermentation product, cell extract, plant extract, animal tissue extract and the like. These compounds may be new compounds or known compounds.

The kit for screening for a compound that alters the bindability of a ligand and the mutant AR of the present invention is a kit, which comprises the mutant AR of the present invention or cells containing the mutant AR of the present invention (animal cells capable of expressing the mutant AR and the like), and the like.

Examples of the screening kit of the present invention are as follow.
1. Reagents for screening
   (1) Buffer for measurement and washing
      Hanks' balanced salt solution (Gibco) supplemented with 0.05% bovine serum albumin (Sigma).
      The solution is sterilized by filtration through a 0.45 µm filter, and stored at 4°C or may be prepared at use.
   (2) Standard mutant AR
      CHO cells expressing the mutant AR of the present invention are passaged in a 12-well plate at a density of 5 x 10⁵ cells/well followed by culturing at 37°C under 5% CO₂ and 95% air for 2 days.
   (3) Labeled ligands
      A solution of DHT labeled with commercially available [³H], [¹²⁵I], [¹⁴C], [³⁵S], etc. is stored at 4°C or -20°C, and diluted to 1 µM with the measurement buffer.
   (4) Standard ligand solution
      DHT is dissolved in and adjusted to 1 mM with PBS containing 0.1% bovine serum albumin (Sigma) and stored at -20°C.
2. Measurement method
   (1) CHO cells expressing the mutant AR of the present invention are cultured in a 12-well culture plate and washed twice with 1 ml of the measurement buffer, and 490 µl of the measurement buffer is added to each well.
   (2) After adding 5 µl of 10⁻³- 10⁻¹⁰ M test compound solution, 5 µl of a labeled ligand is added to the mixture, and the cells are incubated at room temperature for an hour. To determine the amount of the non-specific binding, 5 µl of the non-labeled ligand is added in place of the test compound.
   (3) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)
   (4) The radioactivity is measured using a liquid scintillation counter (manufactured by Beckman Co.), and the percent maximum binding (PMB) is calculated by the equation
      below [Equation 1].
      [Equation 1]
      PMB = [(B - NSB)/(B₀- NSB)] x 100
      PMB: Percent maximum binding
      B: Value obtained in the presence of a test compound
      NSB: Non-specific binding
      B₀: Maximum binding

The compound or its salt, which is obtained using the screening method or the screening kit of the present invention, is a compound that alters the bindability of a ligand and the mutant AR of the present invention. Specifically, it is: (a) a compound that has the mutant AR-mediated cell-stimulating activity (e.g., cell proliferation activity, PSA production, etc.) (so-called agonist for the mutant AR of the present invention); (b) a compound having no cell stimulating-activity (so-called antagonist for the mutant AR of the present invention); (c) a compound that enhances the binding force between the ligand and the mutant AR of the present invention; or (d) a compound that attenuates the binding force between the ligand and the mutant AR of the present invention.

The compound includes a peptide, protein, non-peptide compound, synthetic compound, and fermentation product. It may be a novel or known compound.

Since antagonists to the mutant AR of the present invention can suppress the physiological activities of ligands (androgens) and agonists (some antiandrogen drugs such as bicalutamide and flutamide, and the like) for the mutant AR of the present invention, they are useful as a prophylactic/therapeutic agent for androgen-independent (antiandrogen drug-resistant) cancers, particularly prostate cancer.

A compound that attenuates the binding force between the ligand and the mutant AR of the present invention is useful as a safe and low toxic pharmaceutical agent (e.g., a prophylactic/therapeutic agent for androgen-independent (antiandrogen drug-resistant) cancers, particularly prostate cancer) for decreasing the physiological activities of a ligand (agonist) for the mutant AR of the present invention.

When the compound or salt thereof obtained by using the screening method or the screening kit of the present invention, is used as the aforementioned pharmaceutical composition, the pharmaceutical preparation can be obtained in a conventional manner. For example, in the same manner as the above-described pharmaceutical agent comprising the mutant AR of the present invention, the compound can be made into tablets, capsules, elixirs, microcapsules, sterile solutions, suspensions and the like.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (6) Prophylactic and/or therapeutic agent for various diseases, which comprises a compound that alters the bindability of the mutant AR of the present invention and a ligand (agonist, antagonist)

Since antagonists for the mutant AR of the present invention can suppress the physiological activities for ligands (androgens) and agonists (some antiandrogen drugs such as bicalutamide and flutamide, and the like) for the mutant AR of the present invention, they can be used as a prophylactic/therapeutic agent for androgen-independent (antiandrogen drug-resistant) cancers, particularly prostate cancer. When said compound is used for the above-described diseases, it can be made into a preparation according to a conventional manner.

For example, said compound can be used orally in the form of tablets which may be sugar coated if necessary, capsules, elixirs, microcapsules etc., or parenterally in the form of injectable preparations such as a sterile solution and a suspension in water or with other pharmaceutically acceptable liquids. For example, these preparations can be manufactured by mixing said compound with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80^{™} and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (7) Quantification of the mutant AR of the present invention

The antibody of the present invention is capable of specifically recognizing the AR of the present invention. Therefore, it can be used to quantify the mutant AR of the present invention in a test fluid, especially for quantification by the sandwich immunoassay. That is, the present invention provides, for example, (i) a method of quantifying the mutant AR of the present invention in a test fluid, which comprises competitively reacting the antibody of the present invention with the test fluid and a labeled form of the mutant AR of the present invention, and measuring the ratio of the labeled mutant AR of the present invention bound to the antibody; and, (ii) a method of quantifying the mutant AR of the present invention in a test fluid, which comprises reacting the test fluid with the antibody of the present invention immobilized on a carrier and a labeled form of an antibody of the present invention simultaneously or sequentially, and measuring the activity of the label on the immobilized carrier.

Using monoclonal antibodies against the mutant AR of the present invention (hereinafter also referred to as the monoclonal antibodies of the present invention), the mutant AR of the present invention can be quantified and also detected by tissue staining or the like. For this purpose, an antibody molecule itself may be used, or F(ab')₂, Fab' or Fab fractions of the antibody molecule may also be used. Quantification methods of the mutant AR of the present invention using antibodies of the present invention are not particularly limited. Any assay method can be used, so long as the amount of antibody corresponding to the amount of antigen (e.g., the amount of the mutant AR) in the test fluid, the amount of antigen, or the amount of the complex between antibody and antigen can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H] and [¹⁴C] are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used include fluorescamine and fluorescein isothiocyanate. For the luminescent substance, for example, luminol, luminol derivatives, luciferin, and lucigenin are used. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, insoluble polysaccharides such as agarose, dextran, cellulose, etc.; synthetic resin such as polystyrene, polyacrylamide, silicon, etc., and glass or the like., are included.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the mutant AR of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibodies may be used to increase the measurement sensitivity.

In the methods of assaying the mutant AR of the present invention by the sandwich method, antibodies that bind to different sites of the mutant AR are preferably used as the monoclonal antibodies of the present invention for the primary and secondary reactions.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying the respective immunological methods to the measurement methods of the present invention, any particular conditions or procedures are not required. Systems for measuring the mutant AR of the present invention or its salts are constructed by adding the usual technical consideration in the art to the conventional conditions and procedures. For the details of these general technical means, reference can be made to the reviews and texts [For example, it includes Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Enzymeimmunoassay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing)].

As described above, the mutant AR of the present invention or salt thereof can be quantified with high sensitivity, using the antibodies of the present invention.

Furthermore, by quantifying the mutant AR of the present invention in a living body using the antibody of the present invention, it is possible to diagnose diseases associated with the development/increase of the mutant AR of the present invention, for example, androgen-independent (antiandrogen drug resistant) cancers, particularly prostate cancer. The antibody of the present invention can also be used to specifically detect the mutant AR of the present invention present in a test sample such as body fluid or tissue. Also, the antibody of the present invention can be used to prepare an antibody column used to purify the mutant AR of the present invention, to detect the mutant AR of the present invention in each fraction at the time of purification, to analyze the behavior of the mutant AR of the present invention in the subject cell, and the like.

### (8) Pharmaceutical agent comprising the antibody of the present invention

Neutralizing activity on the mutant AR of the present invention possessed by the antibody against the mutant AR of the present invention means an activity to inactivate a cell-stimulating activity involved by the mutant AR of the present invention.
Therefore, when said antibody has the neutralizing activity, it is possible to inactivate the cell-stimulating activity involved by said mutant AR (e.g., cell proliferation activity, PSA production and the like). Therefore, the antibody of the present invention can be used for the prophylaxis and/or treatment of diseases caused by cell proliferation stimulation by said mutant AR and the like, for example, androgen-independent (antiandrogen drug-resistant) cancers, particularly prostate cancer.

A therapeutic/prophylactic agent for the above-described diseases, which comprises the antibody of the present invention, as a liquid agent as is or as a pharmaceutical composition in an appropriate dosage form, can be administered orally or parenterally to a human or a mammal (e.g., rat, rabbit, sheep, swine, bovine, cat, dog, monkey and the like). The dose varies depending on recipient, target disease, symptoms, route of administration and the like; for example, when the agent is used for the treatment/prophylaxis of an adult cancer patient, normally about 0.01 to about 20 mg/kg body weight, preferably about 0.1 to about 10 mg/kg body weight, more preferably about 0.1 to about 5 mg/kg body weight, per administration, of the antibody of the present invention is conveniently administered by intravenous injection 1 to 5 times or so a day, preferably 1 to 3 times or so a day. In the case of other parenteral administration and oral administration, a dose based thereon can be administered. When the symptom is particularly severe, the dose may be increased according to the symptom.

The antibody of the present invention can be administered as is or as an appropriate pharmaceutical composition. The pharmaceutical composition used in the above-described administration comprises both the above-described antibody or a salt thereof and a pharmacologically acceptable carrier, a diluent or a filler. Such a composition is provided as a dosage form suitable for oral or parenteral administration.

That is, for example, as the composition for oral administration, solid or liquid dosage forms, specifically tablets (including sugar-coated tablets and film-coated tablets), pills, granules, powders, capsules (including soft capsules), syrups, emulsions, suspensions and the like can be mentioned. Such a composition is produced by publicly known methods, and contains a carrier, a diluent or filler normally used in the field of drug formulation. For example, as the carrier or filler for tablets, lactose, starch, sucrose, magnesium stearate and the like can be used.

As examples of the composition for parenteral administration, injections, suppositories and the like are used; the injections include dosage forms such as intravenous injections, subcutaneous injections, intracutaneous injections, intramuscular injections and drip infusion injections. Such an injection is prepared according to a publicly known method, for example, by dissolving, suspending or emulsifying the above-described antibody or a salt thereof in a sterile aqueous or oily solution normally used for injections. As examples of aqueous solutions for injection, physiological saline, an isotonic solution containing glucose or another auxiliary drug, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is normally filled in an appropriate ampoule. Suppositories used for rectal administration are prepared by mixing the above-described antibody or a salt thereof in an ordinary suppository base.

The above-described pharmaceutical composition for oral administration or for parenteral administration is conveniently prepared in a medication unit dosage form suitable for the dosage of the active ingredient. As examples of such a medication unit dosage form, tablets, pills, capsules, injections (ampoules), suppositories and the like can be mentioned; it is preferable that normally 5 to 500 mg, particularly 5 to 100 mg for injections or 10 to 250 mg for other dosage forms, per each medication unit dosage form, of the above-described antibody be contained.

Each of the aforementioned compositions may comprise another active ingredient, as long as no undesirable interaction is produced by formulation with the above-described antibody.

### (9) Pharmaceutical agent comprising an antisense DNA

An antisense DNA capable of complementarily binding to the DNA of the present invention to suppress the expression of said DNA can block a cell-stimulating activity involved by the mutant AR (for example, cell proliferation activity, PSA production and the like) at translational level. Therefore, the antisense DNA can be used for the prophylaxis and/or treatment of diseases caused by cell proliferation stimulation by said mutant AR and the like, for example, androgen-independent (antiandrogen drug-resistant) cancers, particularly prostate cancer.

For example, where the antisense DNA is used, the antisense DNA itself is administered; alternatively, it is inserted into an appropriate vector such as retrovirus vector, adenovirus vector, adenovirus-associated virus vector, etc. and then administered in a conventional manner. The antisense DNA may also be administered per se, or with adjuvants to assist its uptake by gene gun or through a catheter such as a catheter with a hydrogel.

Further, the antisense DNA can be used as an oligonucleotide probe for diagnosis to study the presence or the expression of the DNA of the present invention in tissues or cells.

### (10) DNA-introduced animal

The present invention provides a non-human mammal bearing an exogenous DNA that encodes the mutant AR of the present invention (hereinafter merely referred to as the exogenous DNA of the present invention).

That is, the present invention provides:
(1) A non-human mammal bearing the exogenous DNA of the present invention;
(2) The mammal according to (1), wherein the non-human mammal is a rodent;
(3) The mammal according to (2), wherein the rodent is mouse or rat; and,
(4) A recombinant vector bearing the exogenous DNA of the present invention and capable of expressing in a mammal.

The non-human mammal bearing the exogenous DNA of the present invention (hereinafter simply referred to as the DNA-introduced animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to create the DNA-introduced animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.) or rats (Wistar, SD, etc.), since they are relatively short in ontogeny and life cycle from a standpoint of creating model animals for human disease.

"Mammals" in a recombinant vector that can be expressed in the mammals include the aforementioned non-human mammals and human, etc.
The exogenous DNA of the present invention refers to the DNA of the present invention that is once isolated/extracted from mammals, not the mutant AR gene inherently possessed by the non-human mammals.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the animal cells. For example, in the case of transfecting the human DNA of the present invention, a DNA-introduced mammal that expresses the DNA of the present invention to a high level, can be prepared by microinjecting a DNA construct (e.g., vector, etc.) ligated with the human DNA of the present invention into a fertilized egg of the target non-human mammal downstream various promoters, which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the AR of the present invention, there are *Escherichia coli-*derived plasmids, *Bacillus subtilis*-derived plasmids, yeast-derived plasmids, bacteriophages such as λ phage, retroviruses such as Moloney leukemia virus, etc., and animal viruses such as vaccinia virus, baculovirus, etc. Of these vectors, *Escherichia* coli-derived plasmids, *Bacillus subtilis*-derived plasmids, or yeast-derived plasmids, etc. are preferably used.

Examples of these promoters for regulating the DNA expression include (1) promoters for the DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and chicken β actin promoters etc., which protein can highly express in the whole body, are preferred.

It is preferred that the vectors described above have a sequence for terminating the transcription of the desired mRNA in the DNA-introduced animal (generally called a terminator); for example, a sequence of each DNA derived from viruses and various mammals. SV40 terminator of the simian virus and the like, are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region of the mutant AR of the present invention can be acquired as whole genomic DNA or a portion thereof from a DNA isolated from cells derived from a androgen-independent cancer, particularly prostate cancer, of a human or various mammals (e.g., rabbit, dog, cat, guinea pig, hamster, rat, mouse and the like), or from an antiandrogen drug-resistant cancer cell line obtained by the production method of the present invention described below, alternatively, using a complementary DNA prepared from an RNA derived from the above-described cell (or cell line) by a known method as a raw material. Alternatively, the mutated translational region can be prepared by isolating the translational region of normal AR according to a conventional method, and mutating the same by site-directed mutagenesis.

A non-human mammal having the exogenous DNA of the present invention can be bred under normal breeding conditions as the DNA-bearing animal by confirming the stable retaining of the exogenous DNA via crossing. In addition, the exogenous DNA to be subjected can be utilized as a starting material by inserting the desired exogenous DNA into the plasmid described above. The DNA construct with a promoter can be prepared by conventional DNA engineering techniques. The introduction of the exogenous DNA of the present invention at the fertilized egg cell stage is preserved to be present in all of the germinal and somatic cells of the mammals to be subjected. The fact that the exogenous DNA of the present invention is present in the germinal cells of the animal after DNA transfection means that all of the offspring of the prepared animal have the exogenous DNA of the present invention in all of the germinal and somatic cells. Such an offspring passaged the exogenous DNA of the present invention contains the exogenous DNA of the present invention in all of the germinal and somatic cells. A homozygous animal having the introduced DNA on both of homologous chromosomes can be acquired and then by mating these male and female animals, all the offspring can be bred to have the DNA.

Since non-human mammal having the exogenous DNA of the present invention may express the exogenous DNA of the present invention at a high level, the animal may be the function inactivation type inadaptability of the normal AR by inhibiting the function of the endogenous normal DNA and can be utilized as its disease model animal. For example, using the exogenous DNA-introduced animal of the present invention, it is possible to elucidate the pathologic mechanism of androgen-independent (antiandrogen drug-resistant) cancers, particularly prostate cancer, and to evaluate a method for prophylaxis/treatment of these diseases.

Examples of other potential applications of the DNA-introduced animal of the present invention include:
(1) Use as a cell source for tissue culture;
(2) Elucidation of the relation to a polypeptide that is specifically expressed or activated by the mutant AR of the present invention, by direct analysis of DNA or RNA in tissues of the DNA-introduced animal of the present invention or by analysis of the polypeptide expressed by the DNA;
(3) Research in the function of cells derived from tissues that are cultured usually only with difficulty, using cells of tissue bearing the DNA cultured by a standard tissue culture technique;
(4) Screening of a drug that enhances the functions of cells using the cells described in (3) above; and,
(5) Isolation and purification of the mutant AR of the present invention and preparation of an antibody thereto.

Furthermore, clinical conditions of a disease associated with the AR, including the function inactive type inadaptability of the normal AR can be determined using the DNA-introduced animal of the present invention. Also, pathological findings on each organ in a disease model associated with the AR of the present invention can be obtained in more detail, leading to the development of a new method for treatment as well as the research and therapy of any secondary diseases associated with the disease.

It is also possible to obtain a free DNA-transfected cell by withdrawing each organ from the DNA-introduced animal of the present invention, mincing the organ and degrading with a proteinase such as trypsin, etc., followed by establishing the line of culturing or cultured cells. Furthermore, the DNA-introduced animal of the present invention can serve as identification of cells capable of producing the receptor protein of the present invention, and as studies on association with apoptosis, differentiation or propagation or on the mechanism of signal transduction in these properties to inspect any abnormality therein. Thus the DNA-introduced animal of the present invention can provide an effective research material for the AR of the present invention and for elucidating the function and effect thereof.

To develop a therapeutic drug for the treatment of diseases associated with the AR of the present invention, including the function inactive type inadaptability of the normal AR, using the DNA-introduced animal of the present invention, an effective and rapid method for screening can be provided by applying the method for inspection and the method for quantification, etc. described above. It is also possible to investigate and develop a method for DNA therapy for the treatment of diseases associated with the AR of the present invention, using the DNA-introduced animal of the present invention or a vector capable of expressing the exogenous DNA of the present invention.

The present invention also provides a method of producing an antiandrogen drug-resistant cancer cell line that expresses a mutant AR, which comprises culturing cancer cells sensitive to a specified antiandrogen drug in the presence of said antiandrogen drug, selecting a cancer cell line showing proliferation, analyzing the base sequence of the AR gene in said cancer cell line and selecting a line in which a mutation has occurred in said sequence.

As the starting material cancer cell, there is no limitation, as long as it is sensitive to a specified antiandrogen drug; a cancer cell, preferably a prostate cancer cell, that expresses a normal AR (for example, one having the amino acid sequence represented by SEQ ID NO: 2), or a cancer cell that expresses a mutant AR resistant to a different antiandrogen drug, and the like can be mentioned. Here, "sensitive (resistant) to a specified antiandrogen drug" means being incapable of proliferating (capable of proliferating) in the presence of said antiandrogen drug. As the cancer cell that expresses a normal AR, a biopsy sample obtained from an androgen-dependent cancer patient, a cancer cell derived from an extirpated prostate can be mentioned; as examples of the cell that expresses a mutant AR, a cancer cell derived from a patient who has an androgen-independent cancer relapsing after an endocrine therapy with a different antiandrogen drug, or the LNCaP cell line (expressing the mutant AR represented by T882A) can be mentioned.

Cultivation of a cancer cell can be conducted using an ordinary method used for animal cell culture, with or without appropriate modifications. As examples of the medium, an MEM medium [Science, Vol. 122, 501 (1952)] containing about 5 to 20% fetal bovine serum, DMEM medium [Virology, Vol. 8, 396 (1959)], RPMI 1640 medium [The Journal of the American Medical Association, Vol. 199, 519 (1967)], 199 medium [Proceeding of the Society for the Biological Medicine, Vol. 73, 1 (1950)] and the like can be used. Because cell proliferation is possibly induced by androgens or other steroids contained in the serum, it is preferable that a serum previously treated with dextran-coated charcoal be used. However, to also select AR variations that allow other steroid hormones to act as agonists, use of medium containing a non-treated serum is also preferred. Medium pH is preferably about 6 to 8. Cultivation is normally conducted at about 30°C to 40°C, with aeration and shaking conducted as necessary.

For example, cancer cells are added to any of the above-described media containing 0.0001 to 100 µM of an antiandrogen drug (for example, flutamide, bicalutamide, nilutamide and the like) to obtain a cell density of 1 to 1,000,000 cells/ml, and cultured in a culture vessel such as a multiwell plate under the above-described culture conditions. While conducting medium exchange at an appropriate interval according to a conventional method, the cells are examined for cell proliferation over time. By isolating RNA from a clone that has exhibited cell proliferation, amplifying/isolating AR cDNA using the RT-PCR method with oligonucleotides prepared, for example, on the basis of a known normal AR cDNA sequence as the primers, and analyzing its base sequence by direct sequencing or after subcloning into an appropriate vector, a clone that has actually shown a mutation in the AR is selected.

The mutation site of the mutant AR obtained as described above is characteristic depending on the kind of antiandrogen drug used as the selection pressure; for example, when bicalutamide is used, mutations frequently occur in tryptophan at amino acid number 746 in the amino acid sequence represented by SEQ ID NO: 2 (it is preferably substituted by leucine or cysteine); when flutamide is used, mutations frequently occur in threonine at amino acid number 882 in the amino acid sequence represented by SEQ ID NO: 2 (it is preferably substituted by alanine). These mutation sites agree well with mutation sites that have been clinically found from relapsed cancers to endocrine therapies using respective antiandrogen drugs.

When a cancer cell that expresses a mutant AR (for example, LNCaP cells and the like) is used as the starting material, it is possible to produce a cancer cell line expressing a multiple mutant AR that has a further mutation in a different site. Accordingly, the present invention provides a method of producing an antiandrogen drug-resistant cancer cell line that expresses a multiple mutant AR, which comprises culturing cancer cells that express a mutant AR and are sensitive to a specified antiandrogen drug in the presence of said antiandrogen drug, selecting a cancer cell line showing proliferation, analyzing the base sequence of the mutant AR gene in said cancer cell line and selecting a line that has shown a different mutation in said sequence.

Because an AR is a nuclear receptor having a transcription factor activity to activate the transcription of a particular target gene, agonist or antagonist activity against a mutant AR can easily be assayed by analyzing the expression of the target gene. As the target gene for an AR, a group of genes having a consensus sequence called the androgen-responsive element (ARE) further upstream of the promoter can be mentioned, and PSA and kallikrein are representative examples thereof.

Provided that the cancer cell used is a mammal-derived prostate cancer cell line, a cell line expressing a mutant AR produced by the method of the present invention has an endogenous PSA gene, therefore, agonist or antagonist activity for the mutant AR can be measured and evaluated using an anti-PSA antibody or a DNA encoding PSA or a portion thereof.

Alternatively, also by introducing to the obtained cancer cell line a DNA that encodes a reporter protein under the control of an androgen-responsive promoter (e.g., a promoter having a native sequence corresponding to ARE, such as PSA promoter, or a chimeric promoter comprising a combination of the ARE sequence and a promoter for an animal cell such as SRα promoter, SV40 promoter, LTR promoter, CMV promoter or HSV-TK promoter and the like), and assaying the expression of said reporter protein, agonist or antagonist activity for the mutant AR can be evaluated. The PSA promoter is useful in that it well reflects the behavior of mutant AR in actual prostate cancer cells. When it is necessary to enhance promoter activity, it is preferable that a plurality of (preferably 2 to 5, more preferably 2 or 3) promoters be tandemly ligated. As the reporter protein, luciferase, β-galactosidase, chloramphenicol acetyltransferase, peroxidase and the like can be used. A DNA that encodes a reporter protein can be inserted into the above-described expression vector for an animal cell, and introduced using the above-described gene introduction method.

Because a mutant AR-expressing cancer cell that is resistant to a specified antiandrogen drug (e.g., flutamide) remains sensitive to some other kinds of antiandrogen drugs (e.g., bicalutamide), by culturing a mutant AR-expressing cancer cell line obtained as described above in the presence of a test substance, and examining its action on said mutant AR, a different type of antiandrogen drug capable of suppressing the proliferation of a relapsed cancer that has become resistant to an existing antiandrogen drug can be screened. Accordingly, the present invention also provides a method of screening an antiandrogen drug that exhibits antagonistic action on the mutant AR expressed in a mutant AR-expressing cancer cell line obtained as described above, which comprises culturing said cancer cell line in the presence of a test substance. Although the antagonistic action on mutant AR can also be detected with cell proliferation as an index, it can be detected and evaluated in a shorter time by analyzing the expression of a gene that undergoes transcription activation by said mutant AR. As the gene used, PSA gene described above and a reporter gene under the control of an androgen-responsive promoter (e.g., a promoter having a native sequence corresponding to ARE such as PSA promoter, or a chimeric promoter comprising a combination of the ARE sequence and a promoter for an animal cell such as SRα promoter, SV40 promoter, LTR promoter, CMV promoter or HSV-TK promoter) can be mentioned.

The present invention also provides an antiandrogen drug that exhibits antagonistic action on a mutant AR selected by the above-described method. For example, W746L(C)+T882A, which is a multiple-mutant AR of the present invention, is resistant to both bicalutamide and flutamide, whereas Compound A, which is described in Examples below, exhibits antagonist activity even on a mutant AR on which such existing antiandrogen drugs are ineffective.

When a known antiandrogen drug (for example, bicalutamide, flutamide, and the like) is used in the production method of the present invention for a mutant AR-expressing cancer cell line, an antiandrogen drug-resistant line expressing a mutant AR can be established at latest in about 3 months or so. However, depending on the kind of compound, we can expect a case wherein cell proliferation is not induced even when cultivation is continued for a longer period. Such compounds are useful as antiandrogen drugs of no or little potential for resistant cancer induction. Accordingly, the present invention also provides a method of screening an antiandrogen drug having no or little potential to induce resistant cancer, which comprises culturing cells of an androgen-sensitive cancer in the presence of a test substance, examining over time the appearance of a cancer cell line capable of proliferating under said conditions, and an antiandrogen drug having no or little potential to induce resistant cancer, which is selected by said method.

The present invention provides a prophylactic/therapeutic agent for a hormone-sensitive cancer, preferably prostate cancer, which comprises an antiandrogen drug that exhibits antagonistic action on a mutant AR obtained by the above-described screening method. Because such an antiandrogen drug competitively acts with androgen on both normal and mutant AR, it is capable of suppressing cancer cell proliferation in both the androgen-dependent stage and the androgen-independent stage.

The present invention also provides a prophylactic/therapeutic agent for a hormone-sensitive cancer, preferably prostate cancer, which comprises an antiandrogen drug having no or little potential to induce resistant cancer obtained by the above-described screening method. Because such an antiandrogen drug does not cause a mutation in the AR that confers to resistance to said drug, or is significantly unlikely to cause such mutation compared to other antiandrogen drugs, it is useful in delaying a transition to androgen-independent cancer.

The above-described prophylactic/therapeutic agent can be produced and used according to a commonly used means. For example, in the same manner as the above-described pharmaceutical agent comprising the mutant AR of the present invention, it can be made into tablets, capsules, elixirs, microcapsules, sterile solutions, suspensions and the like.

Since the thus obtained preparation is safe and low toxic, it can be administered to mammals (e.g., human, rat, rabbit, sheep, swine, bovine, cat, dog, monkey and the like).

The dose of the compound or salt thereof varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The present invention also provides an agent for cocktail therapy, which comprises a combination of two or more kinds of antiandrogen drugs that exhibit an antiandrogen action on different mutant ARs. Although a large number of reports concerning AR mutations have been reported to date, there are only a few reported cases of mutant ARs having amino acid mutations at 2 or more sites. Accordingly, provided that two or more kinds of antiandrogen drugs exhibiting antiandrogen action on different mutant ARs (e.g., W746L(C) and T882A) (e.g., bicalutamide and flutamide) are used in combination, even if cancer cells having either mutation have developed, they are unable to proliferate because they are sensitive to either antiandrogen drug, so that transition to the androgen-independent stage can be delayed for a long time.

As the cocktail therapy agent, individual antiandrogen drugs may be made into preparations according to the above-described method, and they may be administered simultaneously, or two or more kinds of antiandrogen drugs may be formulated in one preparation.

The dose of the antiandrogen dugs varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

In performing a cocktail therapy as described above, it is important to realize which mutant AR respective antiandrogen drug is likely to induce. The production method of the present invention for a mutant AR-expressing cancer cell line is useful for such typing of antiandrogen drugs. That is, the typing comprises analyzing mutant ARs obtained by performing said method on various antiandrogen drugs to identify a mutation site that is likely to be induced by the respective antiandrogen drug, and distinguishing antiandrogen drugs (e.g., flutamide, nilutamide and the like) that permit generation of a resistant cancer cell line that expresses the same mutant AR, as one group, from the group consisting of antiandrogen drugs that permit generation of a resistant cancer cell line that expresses a different mutant AR. By combining two or more kinds of optionally chosen antiandrogen drugs classified into different groups by such a classification method, an effective cocktail therapy can be performed.

The information obtained by the above-described classification method of antiandrogen drugs is also useful in conducting a sequential endocrine therapy, wherein one kind of antiandrogen drug is continuously administered until a resistant cancer develops, thereafter another antiandrogen drug classified into a group different from that of said antiandrogen drug is administered to prevent the proliferation of the resistant cancer that has developed.

The present invention also provides a method of evaluating the responsiveness to a specified antiandrogen drug in the transcription factor activity of an AR, which comprises bringing said AR and said antiandrogen drug into contact with mammalian cells containing a gene under the control of PSA promoter that permits expression analysis and analyzing the expression of said gene.

As described above, PSA promoter is useful in that it well reflects the behavior of AR in actual prostate cancer cells. Here, the "PSA promoter" refers to a region containing at least a portion from the essential promoter regions such as TATA box to a sequence corresponding to ARE upstream thereof in the 5' upstream sequence of the prostate-specific antigen gene (in the case of human PSA gene, AGAACAGCAAGTGCT; SEQ ID NO: 3), preferably a region containing a further upstream portion to a 35-base pair portion known as the androgen-responsive region (ARR) [in the case of human PSA gene, GTGGTGCAGGGATCAGGGAGTCTCACAATCTCCTG; SEQ ID NO: 4 (J. Biol. Chem., 271(11): 6379-6388, 1996)], more preferably a region containing about 600 base pairs upstream of the translation initiation site, can be mentioned. Also, when it is necessary to enhance the activity of the PSA promoter, it is preferable that a plurality (preferably 2 to 5, more preferably 2 or 3) of the sequences is tandemly ligated with the above-described region as one unit.

As the "gene that permits expression analysis", a DNA that encodes a reporter protein such as PSA, luciferase, β-galactosidase, chloramphenicol acetyltransferase or peroxidase can be mentioned, as described above for antiandrogen drug screening using a cancer cell line that expresses the mutant AR of the present invention. These DNAs can be inserted, along with PSA promoter, into an expression vector for an animal cell described above (however, lacking a promoter), and introduced to mammalian cells (e.g., COS-7, Vero, CHO, CHO (dhfr⁻), mouse L cells, mouse AtT-20, mouse myeloma cells, rat GH3, human FL cells and the like) using the gene introduction method described above. When the gene that permits expression analysis is PSA gene, the PSA gene, in the form containing the PSA promoter, may be isolated from the genomic DNA of prostate cells using a conventional method, and this may be inserted to and used in an expression vector for an animal cell (however, lacking a promoter). Alternatively, prostate cells having an endogenous PSA gene can also be used as is as "mammalian cells containing a gene under the control of the PSA promoter that permits expression analysis".

The AR applied to the evaluation method of the present invention may be any one; various mammal-derived normal ARs, mutant ARs derived from androgen-independent relapsed cancers, mutant ARs derived from cancer cell lines obtained by the production method of the present invention, recombinant ARs obtained by genetic engineering techniques, and the like can be mentioned. These ARs can be produced by isolation and purification using publicly known methods for purifying a receptor protein. For example, by homogenizing mammalian cells, a resected cancer lesion, the cancer cell line of the present invention, AR-producing host cells and the like, thereafter conducting extraction with an acid and the like, subjecting said extract to a combination of chromatographies such as reversed-phase chromatography and ion exchange chromatography, the AR can be purified and isolated. In the case of a recombinant AR, the AR can be isolated/purified quickly by affinity chromatography by adding a sequence that encodes a tag such as a His tag or GST tag to the AR-coding sequence.

Referring to the AR, one isolated as described above may be added to mammalian cells containing a gene under the control of a PSA promoter that permits expression analysis, it is preferable that said mammalian cells themselves be capable of expressing said AR. As examples of such mammalian cells, host mammalian cells (e.g., COS-7, Vero, CHO, CHO (dhfr⁻), mouse L cells, mouse AtT-20, mouse myeloma cells, rat GH3, human FL cells and the like) into which a gene under the control of the PSA promoter that permits expression analysis and an AR-coding gene are co-introduced, normal mammalian prostate cells, transformed cells obtained by introducing a gene under the control of the PSA promoter that permits expression analysis into a mutant AR-expressing cancer cell line established from a androgen-independent relapsed cancer (e.g., LNCaP cell line) or to a mutant AR-expressing cancer cell line obtained by the production method of the present invention, and the like can be mentioned. In the former case, the AR-coding gene may be inserted to the above-described expression vector for an animal cell and introduced into mammalian cells using the above-described gene introduction method.

As "a specified antiandrogen drug", a substance that exhibits antagonistic action on a known normal or mutant AR (e.g., bicalutamide, flutamide and the like) can be used. "Responsiveness to antiandrogen drug" means how the transcription factor activity of the subject AR is affected by a specified antiandrogen drug. For example, when mammalian cells containing a gene under the control of a PSA promoter that permits expression analysis are brought into contact with the AR derived from the LNCaP-FGC cell line (ATCC Number: CRL-1740) (T882A) or the mutant AR of the present invention (W746L or W746L(C)+T882A) and bicalutamide, expression of said gene is suppressed in the former, whereas the expression is enhanced in the latter. That is, it is judged that T882A is sensitive to bicalutamide, whereas W746L and W746L(C)+T882A are resistant to bicalutamide.

In the above-described method, by using a specified AR and various test substances in place of a specified antiandrogen drug, a modulator of said AR can be screened. "Modulator of AR" means a substance capable of positively or negatively regulating the transcription factor activity of AR, and encompasses both substances having agonist activity on AR and substances having antagonist activity (antiandrogen drugs). As examples of the test substance, a known or new peptide, protein, non-peptide compound, synthetic compound, fermentation product, cell extract, plant extract, animal tissue extract and the like can be mentioned. When mammalian cells containing a gene under the control of a PSA promoter that permits expression analysis are brought into contact with a specified AR and a test substance, and expression of said gene is analyzed, it can be judged that said test substance is a positive modulator of said AR, provided that the expression is enhanced, and that said test substance is a negative modulator (that is, antiandrogen drug) of said AR, provided that the expression is suppressed.

The present invention also provides a kit suitable for use in the above-described evaluation method for the responsiveness to an antiandrogen drug in transcription factor activity of AR or a screening method for an AR modulator. Said kit includes as a component thereof mammalian cells containing a gene under the control of a PSA promoter that permits expression analysis. As said mammalian cells, those prepared by operably linking a DNA that encodes a reporter protein such as PSA, luciferase, β-galactosidase, chloramphenicol acetyltransferase or peroxidase downstream of the PSA promoter, inserting it into the above-described expression vector for an animal cell (however, lacking a promoter), and introducing the vector to mammalian cells (e.g., COS-7, Vero, CHO, CHO (dhfr⁻), mouse L cells, mouse AtT-20, mouse myeloma cells, rat GH3, human FL cells and the like) using the above-described gene introduction method, can be mentioned. When the gene that permits expression analysis is a PSA gene, it may be one obtained by isolating the PSA gene, in the form containing the PSA promoter, from the genomic DNA of prostate cells using a conventional method, inserting it into an expression vector for an animal cell (however, lacking a promoter), and introducing it into mammalian cells in the same manner.

Particularly, when said kit is intended for screening for a modulator of a specified AR, said mammalian cells may further contain an expression vector containing a DNA that encodes said AR. The AR-coding DNA may be inserted into the above-described expression vector for an animal cell, and introduced into mammalian cells using the above-described gene introduction method.

When bases, amino acids and the like are shown in abbreviations in the present specification and drawings, they are based on the abbreviations by the IUPAC-IUB Commission on Biochemical Nomenclature or abbreviations conventionally used in the pertinent field; examples thereof are given below. When an amino acid can have an optical isomer, it is the L-configuration unless otherwise specified.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediamine tetraacetate
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Me: Methyl group
Et: Ethyl group
Bu: Butyl group
Ph: Phenyl group
TC: Thiazolidine-4 (R)-carboxamide group

Also, substituents, protecting groups and reagents often mentioned in the present specification are designated with the following symbols.
Tos: p-Toluenesulfonyl
CHO: Formyl
Bzl: Benzyl
Cl₂Bz1: 2,6-Dichlorobenzyl
Bom: Benzyloxymethyl
Z: Benzyloxycarbonyl
Cl-Z: 2-Chlorobenzyloxycarbonyl
Br-Z: 2-Bromobenzyloxycarbonyl
Boc: t-Butoxycarbonyl
DNP: Dinitrophenol
Trt: Trityl
Bum: t-Butoxymethyl
Fmoc: N-9-fluorenylmethoxycarbonyl
HOBt: 1-Hydroxybenztriazole
HOOBt: 3,4-Dihydro-3-hydroxy-4-oxo-1,2,3-benzotriazine
HONB: 1-Hydroxy-5-norbornane-2,3-dicarboxyimide
DCC: N, N'-dicyclohexylcarbodiimide

Sequence identification numbers in the sequence listing in the present specification indicate the following sequences.

### [SEQ ID NO: 1]

Shows the base sequence of cDNA of wild type human AR.

### [SEQ ID NO: 2]

Shows the amino acid sequence of the wild type human AR.

### [SEQ ID NO: 3]

Shows an ARE sequence in the human PSA promoter.

### [SEQ ID NO: 4]

Shows an ARR sequence in the human PSA promoter.

### [SEQ ID NO: 5]

Shows an oligonucleotide designed to act as a primer for amplifying the human PSA promoter.

### [SEQ ID NO: 6]

Shows an oligonucleotide designed to act as a primer for amplifying the human PSA promoter.

### Free Texts in Sequence Listing

SEQ ID NO: 3: ARE sequence in human PSA promoter.
SEQ ID NO: 4: ARR sequence in human PSA promoter.
SEQ ID NO: 5: Oligonucleotide designed to act as primer for amplifying human PSA promoter.
SEQ ID NO: 6: Oligonucleotide designed to act as primer for amplifying human PSA promoter.

### Examples

The present invention is described in more detail by means of the following Examples, which are not to be construed as limiting the scope of the present invention.

### Example 1: Method of establishing the LNCaP-cxD cell line

When LNCaP-FGC (ATCC Number: CRL-1740) is cultured in a culture broth (RPMI1640 + 10% Dextran Charcoal (DCC)-Fetal Bovine Serum (FBS)) containing 0.1 and 1 µM bicalutamide (commercial name: Casodex), it does not proliferate initially. However, when cultivation was continued for 6 weeks to 13 weeks or more, two cell lines exhibiting proliferation were obtained. These cells were designated LNCaP-cxD11 and LNCaP-cxD2, respectively.

### Example 2: Bicalutamide response of the LNCaP-cxD cell line

LNCaP-cxD11, LNCaP-cxD2 and LNCaP-FGC were sown to 24-well plates at 40000 cells/mL/well, on the following day 0.1-30 µM bicalutamide was added, and 3 days after addition, cells were counted. Also, at this time, the concentration of androgen-dependently produced PSA (Prostatic Specific Antigen) in the culture supernatant was measured.

As a result, the proliferation of LNCaP-cxD11 and LNCaP-cxD2 was significantly promoted by bicalutamide [Fig. 1]. On the other hand, in the parent line LNCaP-FGC, proliferation was significantly suppressed by bicalutamide [Fig. 1]. Also, PSA production was significantly promoted by bicalutamide in LNCaP-cxD11 and LNCaP-cxD2 and significantly suppressed by bicalutamide in the parent line [Fig. 2].

### Example 3: Identification of mutant ARs

After total RNA was extracted from LNCaP-cxD11, LNCaP-cxD2 and LNCaP-FGC, it was converted to cDNA, and the base sequences of the AR genes were analyzed by the PCR direct sequencing method. For LNCaP-cxD11, only a portion, the androgen-binding region, of the AR gene was subjected to sequence analysis.

As a result, in both LNCaP-cxD11 and LNCaP-cxD2 ARs, one mutation in the gene sequence accompanied by an amino acid mutation was present in the androgen-binding region. The TGG (tryptophan) of codon 746 (normally a designation system to write the total number of codons of AR as 919, in which system this codon corresponds to codon 741) was found to be mutated to TTG (leucine) and TGT (cysteine) in LNCaP-cxD11 and LNCaP-cxD2, respectively.

Example 4: Transcription promoting activity of bicalutamide on mutated type AR 5,000,000 cells of Cos-7 were sown to a 150 cm² flask and cultured in a culture broth (DMEM + 10% Dextran Charcoal (DCC)-Fetal Bovine Serum (FBS) + 2mM glutamine) for 24 hours, after which a vector DNA incorporating a wild type AR or a mutated type AR (W741C and W741L type mutations) and a vector DNA having the luciferase gene bound downstream of an androgen-responsive promoter were co-transfected by the liposome method. Two hours later, the medium was replaced with a fresh one and 3 hours of cultivation was conducted, after which 0.01 or 1 µM bicalutamide was added and further 24 hours of cultivation was conducted, after which luciferase activity was measured and the AR transcription activity of bicalutamide was examined.

As a result, bicalutamide exhibited almost no transcription activity on the wild type, whereas bicalutamide exhibited transcription-promoting activity on the W741C and W741L type mutant ARs [Fig. 3].

### Example 5: Screening of compound exhibiting antagonist action on W741C type mutant AR

A compound that inhibits the W741C type mutant AR transcription activity of DHT (dihydrotestosterone), namely a compound that exhibits antagonist action on the W741C type mutant AR was searched for by the same co-transfection system as the assay system described in Example 4; as a result, Compound A [ethyl 2,5-dimethyl-4-(4-nitrophenyl)-1H-pyrrole-3-carboxylate (manufactured by Labotest)] was found [Fig. 4].

### Reference Example 1: Construction of expression vector containing a luciferase gene under the control of a PSA promoter

After genomic DNA was extracted from LNCaP-FGC cells according to a conventional method, a PCR reaction was conducted with this as the template using 5'-GGAGCTCGAATTCCACATTGTTTGCTGCACGTTGG-3' (SEQ ID NO: 5) and 5'-CAAGCTTTGGGGCTGGGGAGCCTCCCCCAGGAGC-3' (SEQ ID NO: 6) as the primers. Using Pyrobest (TaKaRa) as the DNA polymerase, the PCR reaction was conducted in 25 cycles of heating at 94°C for 1 minute, followed by heating at 98°C for 5 seconds, at 59°C for 30 seconds, and at 72°C for 1 minute, with the Gene Amp PCR System 9700 (Applied Biosystems), to yield an about 650 bp DNA fragment containing the PSA (prostate-specific antigen) promoter. After this fragment was cleaved with restriction endonucleases Hind III (TaKaRa) and Sac I (TaKaRa), it was subjected to agarose gel electrophoresis, and a DNA fragment was recovered. The DNA fragment was mixed with the pGL3-Basic vector (Promega), previously digested with Hind III and Sac I, and ligated using the Ligation high (TOYOBO), and *Escherichia coli* DH5α competent cells were transformed, to yield the vector pGL3-PSA-Luc, which has the luciferase gene ligated downstream of the PSA promoter. Furthermore, after pGL3-PSA-Luc was cleaved with Hind III, blunting was conducted using a Blunting kit (TaKaRa), after which the blunt-ended fragment was cleaved with Kpn I (TaKaRa) and a fragment containing the PSA promoter was recovered. After the DNA fragment was cleaved with Sac I, blunting was conducted using a Blunting kit, and the blunt-ended fragment was ligated to Kpn 1-cleaved pGL3-PSA-Luc, and *Escherichia coli* DH5α competent cells were transformed, to yield pGL3-2PSA-Luc, which has two PSA promoters ligated tandem.

### Example 6: Reporter assay system using a PSA promoter

5,000,000 cells of Cos-7 were sown to a 150 cm² flask and cultured in a culture broth (DMEM + 10% Dextran Charcoal (DCC)-Fetal Bovine Serum (FBS) + 2mM glutamine) for 24 hours, after which pGL3-PSA-Luc or pGL3-2PSA-Luc obtained in Reference Example 1 above and a vector DNA incorporating a wild type AR were co-transfected using SuperFect (Qiagen). Two hours later, the medium was replaced with a fresh one and 3 hours of cultivation was conducted, after which 1 µM DHT (5α-dihydrotestosterone) was added and further 24 hours of cultivation was conducted, after which luciferase activity was measured and transcription activity was examined. The results are shown in Fig. 5.

As is evident from Fig. 5, it was found possible to measure the effect of DHT on AR transcription activity using the PSA promoter. Furthermore, it was found possible to obtain more potent activity by tandemly linking the PSA promoter.

### Industrial Applicability

By the method of the present invention, an effective prophylactic/therapeutic agent for androgen-independent relapsed prostate cancer can be screened. Also, development (relapse) of androgen-independent cancers can be detected early. Furthermore, by applying the new endocrine therapy introduced by the present invention, it is expected that the survival rate and period in prostate cancer is significantly improved.

## Claims

1. A method of producing an antiandrogen-drug-resistant cancer cell line that expresses a mutant androgen receptor, which comprises culturing cancer cells sensitive to a specified antiandrogen drug in the presence of said antiandrogen drug, selecting a cancer cell line showing proliferation, analyzing the base sequence of the androgen receptor gene in said cancer cell line and selecting a line in which a mutation has occurred in said sequence, wherein the antiandrogen drug is bicalutamide or an analogue thereof.

2. A method of producing an antiandrogen-drug-resistant cancer cell line that expresses a multiple-mutant androgen receptor, which comprises culturing cancer cells that express a mutant androgen receptor and are sensitive to a specified antiandrogen drug in the presence of said antiandrogen drug, selecting a cancer cell line showing proliferation, analyzing the base sequence of the mutant androgen receptor gene in said cancer cell line and selecting a line that has shown a different mutation in said sequence, wherein the antiandrogen drug is bicalutamide or an analogue thereof.
